# EUROPÄISCHE PATENTSCHRIFT

(11) **EP 3 068 341 B1**
(45) Veröffentlichungstag und Bekanntmachung des Hinweises auf die Patenterteilung: **28.08.2019**
(21) Anmeldenummer: 14806164.1
(22) Anmeldetag: 17.11.2014
(51) Int. Cl.: A61F 2/16

(54) **VORRICHTUNG ZUR AUFNAHME EINER INTEROKULAREN LINSE**
DEVICE FOR RECEIVING AN INTRAOCULAR LENS
DISPOSITIF DESTINÉ À RECEVOIR UNE LENTILLE INTRAOCULAIRE

(30) Priorität: 15.11.2013 CH 19112013; 17.12.2013 CH 20842013
(43) Veröffentlichungstag der Anmeldung: 21.09.2016
(73) Patentinhaber: Medicel AG, 9423 Altenrhein (CH)
(72) Erfinder: DOCKHORN, Volker, CH-9305 Berg (CH); HOHL, Emil, CH-9434 Au (CH)
(74) Vertreter: Riederer Hasler & Partner Patentanwälte AG
(86) Internationale Anmeldenummer: PCT/CH2014/000166
(87) Internationale Veröffentlichungsnummer: WO 2015/070358

(56) Entgegenhaltungen:
- EP-A1- 1 905 386
- EP-A1- 2 286 762
- WO-A2-03/044946
- WO-A2-2008/098384
- FR-A1- 2 892 920
- JP-A- 2006 068 440

## Beschreibung

### TECHNISCHES GEBIET DER ERFINDUNG

Die vorliegende Erfindung betrifft eine Vorrichtung zur Aufnahme einer intraokularen Linse.

### HINTERGRUND DER ERFINDUNG

Bei Kataraktoperationen werden heutzutage standardmässig künstliche Linsen, sogenannte Intraokularlinsen, in den Kapselsack des Auges eingesetzt.

Bei der Operation wird eine okulare Inzision von typischerweise 2 bis 4 mm gemacht, durch welche die natürliche Augenlinse zunächst entfernt und dann das Implantat eingesetzt wird. Zum Einsetzen wird die künstliche Linse in gefaltetem Zustand durch die Inzision in den Kapselsack eingeführt. Sobald die gefaltete Linse in den Kapselsack eingeführt ist, entfaltet sich diese wieder in ihre ursprüngliche Form.

Die heute üblichen künstlichen Linsen bestehen aus einem optischen Linsenkörper und zwei oder mehreren von diesem quer zur optischen Achse des Linsenkörpers peripher abstehenden Haptiken, welche im Kapselsack als Positionsfedern für den Linsenkörper dienen. Beispielsweise stehen zwei Haptiken, welche am Linsenkörper einander gegenüberliegend angeordnet sind, gleichgesinnt spiralierend vom Linsenkörper ab.

Verbesserte Operationswerkzeuge und Implantate ermöglichen es den Chirurgen, die Inzisionen zusehends kleiner zu machen. Die Entfernung der natürlichen Augenlinse kann heutzutage bereits durch Inzisionen von weniger als 2 mm erfolgen. Dies macht jedoch nur Sinn, wenn auch die intraokulare Linse durch eine derartig kleine Inzision eingesetzt werden kann.

Zum Einsetzen einer intraokularen Linse sind in den vergangenen Jahren Linsenträger oder Kartuschen entwickelt worden, in welche eine Linse ladbar und sodann mittels eines Injektors aus dem Linsenträger ausgestossen werden kann.

Beispiele für solche Linsenträger oder Kartuschen und Injektoren sind zum Beispiel aus den Patentschriften US 6 267 768, US 5 810 833, US 6 283 975, US 6 248111, US 4 681 102, US 5 582 614, US 5 499 987, US 5 947 975, US 6 355 046 und EP 1 290 990 B1, sowie den Offenbarungen US 2004/0199174 A1, EP 1905 386 A1 und WO 03/045285 A1 bekannt.

Bei der Injektorvorrichtung gemäss der US 4 681 102 sind die Kartusche, die als Faltvorrichtung für die Linse ausgebildet ist, und die Injektordüse separate Teile. Die Kartusche kann in das Injektorgehäuse eingeschoben werden, worauf die Injektordüse vorn am Injektorgehäuse aufgeschraubt werden kann.

Bei der Injektorvorrichtung gemäss der US 5582614 und den meisten vorbekannten Injektorvorrichtungen, wie z.B. US 6 267 768, US 5 810 833, US 6 283 975 und US 6 248 111, besteht die Kartusche einstückig aus einer Faltvorrichtung und einer Injektordüse.

Intraokularlinsen werden vom Hersteller steril verpackt und gegebenenfalls in einem-Flüssigkeitsbad geliefert. Je nach Linsenmaterial kann die Lagerung in einer Flüssigkeit notwendig sein, um die Linse vor Austrocknung zu schützen. Bei der Operation muss die Linse im sterilen Bereich der Verpackung entnommen und in eine Kartusche oder direkt in einen Injektor eingesetzt bzw. geladen werden. Da diese Linsen sehr kleine und federnde Gebilde darstellen, besteht beim Bestücken der Kartusche bzw. des Injektors die Gefahr, dass die Linse fallengelassen wird oder beim Falten wegspringt und dabei ihrer Sterilität verlustig geht. Zudem sind die Linsen sehr empfindliche Gebilde, welche beim Falten, Injizieren und Umladen in eine Kartusche leicht beschädigt werden können. Die Beschädigungsgefahr ist insbesondere gross für die sogenannte Haptik, welche den optischen Teil der Linse umgibt. Es ist auch möglich, dass der optische Teil beispielsweise durch die Pinzette beim Einsetzen in die Kartusche beschädigt wird. Diese Gefahren sind besonders gross bei Kartuschen gemäss der US 4 681 102, welche keine Massnahmen zum Erfassen der Linsenränder beim Falten vorsieht. Demgegenüber schlägt die US 5 582 614 vor, bei den Kartuschen an den freien Enden ihrer Halbschalen Rillen anzubringen, welche beim Falten der Linse die Linsenränder erfassen. Die US 5 499 987 sieht eine besondere Ausbildung der Rillen vor. Es wird nämlich vorgeschlagen, die Rillen gegen die Düse hin weniger tief zu gestalten, um so deren Transport in die Düse zu erleichtern (Fig. 34, 39, 40 und 41 in US 5 499 987). Nachteilig ist jedoch, dass Kartuschen mit solchen Rillen die Linsenränder nicht sicher erfassen können, wenn die Linse einen relativ grossen Durchmesser aufweist und die Kartusche zum Einlegen der Linse weit geöffnet werden muss, also ein grosser Öffnungswinkel besteht. Diesen Nachteil weist die Kartusche gemäss der US 5 947 975 oder der US 6 355 046 nicht auf, denn sie besitzt zwei Scharniere, so dass sich eine andere Faltgeometrie ergibt als bei Kartuschen mit einem einzigen Scharnier. Nachteilig ist jedoch, dass für jede Linsengrösse eine dieser Grösse entsprechende Kartusche notwendig ist. Ein weiterer Nachteil besteht darin, dass die Faltung der Linse nicht optimal ist (Fig. 11 und Fig. 15 in US 5 947 975 oder US 6 355 046), so dass der Durchmesser der Injektorspitze relativ gross gehalten werden muss. Deswegen ist auch eine unerwünscht grosse okulare Inzision notwendig, um die Linse in den Kapselsack des Auges einzuführen.

Wie die EP 1 290 990 B1, WO 03/045285 A1 und EP 1905 386 A1 zeigen, wurde in späteren Jahren weiterhin an der ursprünglichen Kartuschenkonstruktion festgehalten, welche zwei durch ein einziges Scharnier verbundene Halbschalen aufweist, sei es mit oder ohne Rillen oder Halteeinrichtung zum Erfassen der Linsenränder.

Die Offenbarung WO 03/045285 A1 zum Beispiel zeigt ein Verfahren zum Einführen einer intraokularen Linse in den Kapselsack des Auges, bei welchem ein Überdruck erzeugt wird, um eine in einem Gleitmittel schwimmend aufgenommene Linse aus der Injektordüse auszustossen. Ein kompressibler und deformierbarer Kolben passt sich dem sich nach vorn verengenden Düsenkanal kontinuierlich an. Die Linse wird auf ihrem Weg weiter gefaltet und hat am Ende ihres Weges einen sehr kleinen Durchmesser. Aufgrund der Deformierbarkeit des Kolbens, kann das Ende des Düsenkanals sehr eng gehalten sein, folglich ist lediglich eine sehr kleine Inzision nötig. Ein Set zur Ausführung des Verfahrens enthält einen Linsenträger und eine Linse. Die Linse befindet sich in spannungsfreiem Zustand im Linsenträger. Linse und Linsenträger sind vorzugsweise von einem Halter getragen und bis zur Verwendung in einer Packung steril verpackt, und zwar im Fall einer hydrophilen Linse in einer Flüssigkeit, welche die Linse vor dem Austrocknen schützt. Bei der Operation wird der Linsenträger samt der darin gelagerten Linse der Packung entnommen, in den Injektor eingesetzt und gefaltet. Hierauf wird durch den Kanal eine Gleitflüssigkeit eingefüllt. Die Linse kann nun in den Kapselsack des zu behandelnden Auges injiziert werden.

Weil Kartuschen mit Rillen zum Erfassen der Linsenränder bei der Faltung nicht befriedigten, wird in der US 2004/0199174 eine Kartusche vorgeschlagen, die ebenfalls in herkömmlicher Weise zwei Halbschalen aufweist. Darin wird insbesondere eine Injektorvorrichtung gezeigt, bei welcher das Injektorgehäuse aus einem Zylinder zur Aufnahme des Kolbens, einer Faltvorrichtung für die Linse und einer Injektordüse besteht. Zusätzlich ist ein flexibles, elastisches Band vorgesehen, das von der ersten Halbschale zur zweiten Halbschale führt und durch einen Schlitz an deren Kante durchgezogen werden kann. Eine intraokulare Linse wird zwischen einer Schlaufe des Bandes und den Halbschalen eingeführt. Durch Zug am Band können die Halbschalen aufeinander zu bewegt werden und wird die Linse (welche ungefaltet über den Rand der Halbschalen hinausragt) in die Halbschalen hineingedrückt, sodass dabei die intraokulare Linse gefaltet wird. Beim Falten wird somit die Linse durch das Band gehalten. Das Band wirkt als Falthilfe. Nachteilig ist die aufwendige Konstruktion der Kartusche und deren komplizierte Handhabung. Zudem kann die Haptik z.B. zwischen Band und Schlitz eingeklemmt werden.

Es gibt vorgeladene Systeme. Diese werden bereits in der Fabrik mit einer Linse in ungefaltetem Zustand geladen (sogenannte pre-loaded systems, d.h. vorgeladene Systeme), so dass der bisherige heikle Ladevorgang vor der Operation nicht mehr nötig ist. Der Operateur braucht nur noch den Faltvorgang vorzunehmen und die Gleitflüssigkeit einzuführen. Für vorgeladene Systeme sind Halterungen bekannt, welche eine vorgeladene Linse während dem Transport in Ihrer Lagerposition halten. Diese Halterungen werden, bevor die Linse in ein Auge injiziert werden kann, entfernt oder fallen beim Falten der Linse ab. Das Entfernen einer Halterung stellt einen zusätzlichen Manipulationsschritt dar. Ein abfallendes Teil kann sich störend auswirken; zum Beispiel indem es aufgefangen und aus dem Operationsbereich entfernt werden muss.

Im Allgemeinen können im Stand der Technik Systeme für vorgefaltete Linsen und Systeme für nicht vorgefaltete Linsen unterschieden werden. Bei den Systemen ohne vorgefaltete Linsen, werden die Linsen erst während dem Stossprozess zur Injektion gefaltet. Hierfür werden insbesondere Systeme verwendet, bei welchen die Linse von Hinten, noch unverformt bzw. ungefaltet in eine Ladekammer geladen wird (wie dies z.B. in US 5,810,833 gezeigt wird). Systeme für nicht vorgefaltete Linsen benötigen nachteiligerweise relativ grosse okulare Inzisionen, weil beim Stossprozess aus verschiedenen Gründen nicht beliebig klein eingefaltet werden kann. Bei den Systemen mit vorgefalteten Linsen, werden die Linsen vor dem Stossprozess zur Injektion gefaltet bzw. vorgefaltet. Hierfür werden insbesondere Flügelkartuschen (wie z.B. in US 6,267,768, US 6,248,111, US 5,947,975 oder US 4,681,102 offenbart) verwendet. Die Linse wird beim Schliessen der Flügelkartusche gefaltet und liegt in der Ladekammer in vorgefaltetem Zustand vor. Systeme mit Flügelkartuschen benötigen vorteilhafterweise nur kleine Inzisionen. Nachteilig ist, dass die Linse, insbesondere deren Haptik, beim Einlegen und Falten eingeklemmt werden oder die Haptik dabei eine ungünstige Lage einnehmen kann.

Keines der vorgehend erwähnten Dokumente offenbart ein System, welches immer fehlerfrei manipulierbar ist und/oder das Risiko, die Haptik der Linse beim Falten einzuklemmen, völlig unterbindet. Eine eingeklemmte oder unvorteilhaft eingeschlossene Haptik kann spätestens beim Injizieren der Linse brechen. Sind viele Manipulationsschritte zur Vorbereitung von Linse und Injektor nötig, können diese entsprechend viele Fehlerquellen darstellen.

WO 2008/098384 offenbart eine Vorrichtung gemäß dem Oberbegriff des Anspruchs 1.

### AUFGABE

Es ist daher Aufgabe der vorliegenden Erfindung eine Vorrichtung zum einfachen Beladen mit einer intraokularen Linse zu schaffen, welche die Nachteile der beschriebenen bekannten Systeme und Methoden vermeidet. Zudem soll eine Vorrichtung geschaffen werden, welche eine intraokulare Linse faltet, ohne diese beim Falten und/oder Injizieren zu beschädigen und zur Verwendung mit kleinen Inzisionen geeignet ist. Im Weiteren soll eine Vorrichtung bereitgestellt werden, welche hinsichtlich der Manipulationsschritte zur Vorbereitung von Linse und Injektor optimiert ist. Insbesondere sollten möglichst wenige Manipulationsschritte nötig sein, welche nach Anlieferung von Linse und Injektor bzw. welche unmittelbar vor dem chirurgischen Eingriff an der Vorrichtung vorgenommen werden müssen. Dadurch sollen Fehlerquellen reduziert werden. Ein weiteres Ziel ist es eine Vorrichtung zu schaffen welche in der Anwendung lediglich kleine Inzisionen im Auge benötigt.

Diese und andere Ziele werden durch die Merkmale des Patentanspruchs 1 erreicht. Weiterbildungen und/oder vorteilhafte Ausführungsvarianten der Erfindung sind Gegenstand der abhängigen Patentansprüche.

### ZUSAMMENFASSUNG DER ERFINDUNG

Erfindungsgemäß wird die Aufgabe durch eine Vorrichtung nach den Merkmalen des Anspruchs 1 gelöst. Die Aufgabe wird auch durch eine Vorrichtung zur Aufnahme einer intraokularen Linse mit einer ersten und einer zweiten Halbschale gelöst, welche durch ein erstes Gelenk gelenkig miteinander verbunden sind und relativ zueinander von einer offenen Stellung in eine geschlossene Stellung bewegt werden können, wobei die Halbschalen in der offenen Stellung eine offene Kammer bilden, und die Halbschalen in der geschlossenen Stellung eine umschlossene Kammer bilden, und wobei längsseitig an der ersten der beiden Halbschalen ein Deckelglied verschwenkbar angeordnet ist, welches in der offenen Stellung die offene Kammer begrenzt, insbesondere überdeckt bzw. überspannt, und in der geschlossenen Stellung ausserhalb (bzw. im Wesentlichen ausserhalb) der umschlossenen Kammer, insbesondere zwischen den beiden Halbschalen, liegt bzw. positioniert ist. Das Deckelglied ist (und vorzugsweise bleibt) in offener Stellung sowie in geschlossener Stellung an der ersten Halbschale angeordnet bzw. mit der ersten Halbschale verbunden.

Vorteilhafterweise ist die erfindungsgemässe Vorrichtung als Kartusche zum Einsetzen in ein Injektorgehäuse ausgebildet. Alternativ kann die erfindungsgemässe Vorrichtung ein integrierter Teil eines Injektors sein.

Mit der genannten Vorrichtung kann ein Einklemmen der Linse und insbesondere ihrer Haptik beim Schliessen der Halbschalen vermieden werden. Bei Verwendung der erfindungsgemässen Vorrichtung, ist für den Nutzer visuell erkennbar, wo die Linse in der erfindungsgemässen Vorrichtung bzw. Kartusche positioniert ist. Die Ausgangsposition der Linse vor dem Faltvorgang kann somit visuell geprüft werden. Ein fehlerhaftes Einlegen der Linse kann ausgeschlossen werden. Das Deckelglied wirkt demgemäss auch als Positionierhilfe und als Abdeckung während der Lagerung und Bereithaltung der Linse.

Die Erfindungsgemässe Vorrichtung kann in der Anwendung mit relativ kleinen Inzisionen (insbesondere bei Inzisionen mit einem Durchmesser von weniger als 2.5 mm, bevorzugt weniger als 2.2 mm, weiter bevorzugt weniger als 2 mm, weiter bevorzugt weniger als 1.5 mm) am Auge verwendet werden. Dies gelingt, weil die erfindungsgemässe Vorrichtung eine mantelartig umschliessende und somit (längsseitig) geschlossene Ladekammer für eine zu injizierende, gefaltete Linse aufweist. Die Linse ist darin auf einen besonders kleinen Querschnittsdurchmesser vorgefaltet und kann mittels einer engen Kanüle und durch eine - wie beschrieben - besonders kleine Inzision injiziert werden. Besonders vorteilhaft wird die genannte Vorrichtung mit einem verformbaren Stempel, insbesondere einem Silikonstempel (z.B. gemäss der Offenbarungsschrift WO 03/045285 A1), verwendet.

Die im Folgenden angeführten vorteilhaften Ausführungsmerkmale führen allein oder in Kombination miteinander zu weiteren Verbesserungen der erfindungsgemässen Vorrichtung und ihrer Anwendung.

Verschwenkbar angeordnet kann bedeuten, dass das Deckelglied an der ersten Halbschale angeformt ist. Herstellungstechnisch ist insbesondere vorteilhaft, wenn das Deckelglied zusammen mit der erste Halbschale und gegebenenfalls weiter zusammen mit der zweiten Halbschale einstückig ausgestaltet ist. Eine einstückige Kartusche kann z.B. durch Spritzguss kostengünstig hergestellt werden.

Das Deckelglied ist insbesondere dadurch verschwenkbar, dass es zweckmässigerweise selbsttragend, steif und/oder plattenförmig ausgebildet ist.

Vorteilhafterweise sind zumindest die erste Halbschale und das Deckelglied, vorzugsweise die erste Halbschale, die zweite Halbschale und das Deckelglied nicht lösbar miteinander verbunden; d.h. unter Bedingungen des normalen Gebrauchs nicht lösbar. Der normale Gebrauch beinhaltet hierbei insbesondere das Aufladen, Lagern, Falten und Ausstossen bzw. Injizieren einer Linse.

Das Deckelglied ist insbesondere am längsseitigen Rand der ersten Halbschale, d.h. auf der dem ersten Gelenk abgewandten Längsseite der ersten Halbschale, verschwenkbar angeordnet.

Zweckmässigerweise ist das Deckelglied über ein zweites Gelenk, welches z.B. als Scharnier, insbesondere als Filmscharnier, ausgebildet ist, mit der ersten Halbschale verbunden. Das Deckelglied ist insbesondere um das zweite Gelenk verschwenkbar.

Eine Biegerille kann auf der der Innenfläche der ersten Halbschale abweisenden Deckelgliedfläche ausgebildet sein.

Zur Stabilisierung in offener Stellung der Vorrichtung ist vorteilhaft, wenn am längsseitigen Rand der zweiten Halbschale eine Deckelgliedauflage, bevorzugt mit Einrastrille, ausgebildet ist.

Gegebenenfalls ist jede Halbschale mit einer Auflage ausgestattet. Die Auflage kann z.B. in Form zumindest einer Gleitschiene ausgebildet sein.

Die Vorrichtung ist vorteilhafterweise derart ausgebildet, dass beim Schliessen der beiden Halbschalen das Deckelglied über den längsseitigen Rand der zweiten Halbschale aus der sich schliessenden Kammer hinaus rutscht.

Zur Gewährleistung der Funktion als Ladekanal bildet die umschlossene Kammer in der geschlossenen Stellung der Halbschalen einen Kanal, insbesondere einen zylindrischen Ladekanal. Jede der Halbschalen bildet dabei vorteilhafterweise ein zylindrisches Segment.

Zur besseren Handhabung sind an beiden Halbschalen, insbesondere am jeweiligen längsseitigen Rand der Halbschalen, Flügel (d.h. Flügelgriffe) angeordnet.

Von Vorteil kann sein, wenn an den Flügeln ein Verschluss, insbesondere ein Schnappverschluss, ausgebildet ist.

In geschlossener Stellung der Halbschalen ist das Deckelglied ausserhalb (bzw. im Wesentlichen ausserhalb) der umschlossenen Kammer angeordnet, vorzugsweise liegt es (zumindest teilweise) zwischen den Rändern der Halbschalen bzw. (im Wesentlichen) zwischen den Flügeln (bzw. zwischen den Rändern der Halbschalen, welche als Flügel ausgebildet sind), gegebenenfalls kann es zwischen den Rändern bzw. den Flügeln eingeklemmt sein.

Das Deckelglied ist vorzugsweise selbsttragend bzw. formstabil ausgeführt, insbesondere als Plättchen. Das Deckelglied ist insbesondere unter Bedingungen des normalen Gebrauchs formstabil bzw. selbsttragend. Der normale Gebrauch der Vorrichtung beinhaltet hierbei insbesondere das Aufladen, Lagern, Falten und Ausstossen bzw. Injizieren einer Linse. Das Deckelglied ist unter normalem Gebrauch also nicht biegbar, d.h. nicht in sich biegbar, verformt sich somit nicht wesentlich, und verhält sich im Wesentlichen steif. Dadurch ist das Deckelglied insbesondere um das zweite Gelenk verschwenkbar. Ein Band (das definitionsgemäss biegbar ist) eignet sich nicht als Deckelglied.

Das Deckelglied kann einen Durchbruch aufweisen, welcher zur Befüllung der noch offenen Kammer sowie gegebenenfalls der Injektordüse mit Gleitmittel dient.

Nützlich ist, wenn das Deckelglied einen Stopper für die Linse aufweist. Der Stopper ist vorzugsweise vorrichtungsvorderseitig bzw. kartuschenvorderseitig am Deckelglied angeordnet, insbesondere angeformt. Zweckdienlicherweise wird die Linse inklusive deren vordere Haptik durch den Stopper daran gehindert nach vorne auszutreten und gegebenenfalls aus der Vorrichtung herauszufallen. Ferner lässt sich mittels dieses Stoppers, die vordere Haptik gegen die Optik platzieren, wenn die Linse nach vorne gegen den Stopper gedrückt wird. Hierdurch ergibt sich ein besseres Austrittsverhalten der Linse.

Insbesondere kann nützlich sein, wenn das Deckelglied zwei Stopper, d.h. einen ersten Stopper und einen zweiten Stopper aufweist, den ersten Stopper (wie oben aufgezeigt) zur Begrenzung der vorderen Haptik und den zweiten Stopper zur Begrenzung der hinteren Haptik. Diese Ausführung mit zwei Stopper ist insbesondere dadurch vorteilhaft, dass eine in Flüssigkeit gelagerte Linse, welche sich vorgeladen (pre-loaded) in einer Vorrichtung, welche als Kartusche ausgebildet ist, befindet, aus dem Aufbewahrungsbehälter entnommen und in den Injektor eingesetzt werden kann, ohne dass die Linse dabei über eine der beiden Seiten der Kartusche (d.h. die vordere Stirnseite oder die hintere Stirnseite der Kartusche) herausfallen kann. Vorteilhafterweise ist das Deckelglied gegenüber den Halbschalen in Längsrichtung verkürzt ausgeführt, vorzugsweise verkürzt und gestuft oder verkürzt und eingeschlitzt.

In Übereinstimmung mit einem Injektorgehäuse kann an einer der Halbschalen eine Steckvorrichtung zum Einstecken in eine Aufnahmeöffnung des Injektorgehäuses ausgebildet sein. Vorzugsweise ist die Steckvorrichtung an der zweiten Halbschale vorgesehen.

Herstellungstechnisch ist es von Vorteil, wenn die Vorrichtung einstückig ist und vorzugsweise aus Kunststoff besteht. Spritzgusstechnik kann in diesem Fall angewendet werden.

Im Weiteren ist hierin offenbart ein Injektor mit einem Injektorgehäuse und einem im Injektorgehäuse längsverschiebbaren Stössel zur Verwendung mit einer als Kartusche ausgebildeten Vorrichtung wie sie oben beschrieben ist.

Die oben genannte Aufgabe wird durch ein Verfahren zum Falten einer intraokularen Linse gelöst, welches die folgenden Schritte beinhaltet:
- Bereitstellen eines Hohlraums dessen Ummantelung zumindest eine erste Halbschale, eine zweite Halbschale und ein Deckelglied beinhaltet, wobei die erste Halbschale auf einer Seite über ein erstes Gelenk mit der zweiten Halbschale gelenkig verbunden ist und auf der gegenüberliegenden Seite über ein zweites Gelenk mit einem Deckelglied gelenkig verbunden ist, wobei das Deckelglied mit dem (freien) Rand der zweiten Halbschale gleitend in Kontakt ist,
- Einführen einer Linse in den Hohlraum, indem der gesamte Linsenkörper (41) der Linse (33) in den Hohlraum eingebracht wird,
- Zusammenführen der beiden Halbschalen über das erste Gelenk, indem die Seite der ersten Halbschale mit dem Deckelglied durch Drehung um das erste Gelenk dem Rand der zweiten Halbschale zugeführt wird, wobei gleichzeitig das Deckelglied über den Rand der zweiten Halbschale aus einem sich zwischen den beiden schliessenden Halbschalen bildenden Hohlraum gleitet, was gleichzeitig eine Drehung des Deckelglieds um das zweite Gelenk bedeutet. Das Deckelglied gleitet insbesondere zwischen zwei Flügeln, welche an je einer der Halbschalen ausgebildet bzw. angeformt sind.

Die genannte Ummantelung ist zumindest dreigliedrig bzw. dreiteilig beinhaltend eine erste Halbschale, eine zweite Halbschale und ein Deckelglied. Die drei Glieder bzw. Teile sind dabei vorzugsweise integrale Teil eines einzigen Spritzgussteils.

Vorzugsweise wird zumindest eine Haptik der Linse, zum Linsenkörper hin gestossen. Insbesondere wird die vordere Haptik der Linse, mittels eines Stoppers zum Linsenkörper hin gestossen. Bei vorhandenem vorderen Stopper kann unter Stösseldruck auf die Linse die vordere Haptik gegen den Linsenkörper gedrückt werden. Weiter kann es vorteilhaft sein, die hintere Haptik der Linse, direkt über einen Stössel (d.h. mittels Stössel), insbesondere einen flexiblen Stössel, zum Linsenkörper hin zu stossen. Auf diese Weise werden die Haptiken vorgefaltet.

Vorteilhaft ist, dass die Linse in spannungslosem Zustand in den Hohlraum eingeführt werden kann. Das heisst, die Linse kann ohne von aussen angelegter mechanischer Spannung, insbesondere ohne Verbiegung oder Faltung der Linse, in die Vorrichtung (insbesondere per Hand) eingelegt werden.

Zweckdienlicherweise liegt die Linse - insbesondere nach dem Einlegen per Hand - (zumindest teilweise) auf den Innenflächen der beiden Halbschalen auf. Das heisst, dass die Linse zumindest auf der Innenfläche jeder Halbschale an einem Punkt aufliegt.

Zweckmässigerweise wird bzw. ist die Linse an ihren Rändern durch die beiden Halbschalen eingefasst und wird mit den Halbschalen zusammen (vorzugsweise in ungefähr gleicher Richtung) gefaltet. Das Deckelglied ist beim Faltvorgang nicht mit der Linse in Kontakt bzw. berührt diese nicht. Das Deckelglied wirkt bei der Faltung nicht auf die Linse ein, ist also keine Falthilfe.

Vorzugsweise wird beim Zusammenführen der beiden Halbschalen bis zur gegenseitigen Berührung bzw. bis zum gegenseitigen Aneinanderstossen der beiden längsseitigen Ränder der beiden Halbschalen zusammengeführt, wobei das Deckelglied eingeklemmt wird. Das Deckelglied kann zwischen den beiden längsseitigen Rändern geklemmt werden. Vorteilhaft sind die Ränder mit Flügeln bestückt, welche bei geschlossener Stellung der Halbschalen das Deckelglied klemmen und dadurch sichern und fixieren. Es wird sichergestellt, dass das Deckelglied aus der sich schliessenden Kammer ausgleitet und gleichzeitig ausserhalb der sich schliessenden Kammer fixiert wird, insbesondere zwischen den Rändern der Halbschalen vorzugsweise zwischen den Flügeln, sodass das Deckelglied beim nachfolgenden Ausstossen der Linse weder in der umschlossenen Kammer noch ausserhalb dieser den Ausstoss der Linse, z.B. zur Injektion in ein Auge, behindern kann. Das verschwenkbare Deckelglied gleitet vorzugsweise beim Verschwenken am zweiten Flügel entlang und aus den sich formenden schliessenden Halbschalen.

Im Weiteren ist offenbart eine erfindungsgemässe Vorrichtung zur Aufnahme einer intraokularen Linse mit einer ersten und einer zweiten Halbschale, welche durch ein erstes Gelenk gelenkig miteinander verbunden sind und relativ zueinander von einer offenen Stellung in eine geschlossene Stellung bewegt werden können, wobei die Halbschalen in der offenen Stellung eine offene Kammer bilden, welche zum Einlegen einer Linse dient, und die Halbschalen in der geschlossenen Stellung eine umschlossene Kammer bilden, welche eine Ausstosspassage definiert und zum Ausstossen der Linse dient, welche Vorrichtung sich erfindungsgemäss, zumindest dadurch auszeichnet, dass an der ersten der beiden Halbschalen mindestens ein Stopper (bzw. Stopperelement) verschieblich oder verschwenkbar angeordnet ist, welcher in der offenen Stellung die offene Kammer in Ausstossrichtung begrenzt und in der geschlossenen Stellung ausserhalb (bzw. im Wesentlichen ausserhalb) der umschlossenen Kammer seitlich der Ausstosspassage positioniert ist. Der Stopper hilft beim Bestücken der Vorrichtung mit einer Linse, die Linse und deren Haptik richtig zu positionieren.

Diese Vorrichtung kann mit den Merkmalen, wie sie oben definiert sind, kombiniert werden, insoweit sich die Merkmale nicht gegenseitig ausschliessen. Insbesondere kann das Merkmal eines Deckelglieds in Kombination mit dem mindestens einen Stopper vorhanden sein.

Der Stopper (bzw. das Stopperelement) ist zweckmässigerweise an einem Halteelement, z.B. dem Deckelglied oder einem Arm, ausgebildet, wobei das Halteelement zweckmässigerweise in der offenen Stellung die offene Kammer überbrückt bzw. überspannt und in der geschlossenen Stellung (im Wesentlichen) ausserhalb der umschlossenen Kammer positioniert ist.

Vorteilhaft ist, dass das Halteelement am längsseitigen Rand der ersten Halbschale verschieblich bzw. verschwenkbar angeordnet ist. Durch diese verschiebliche bzw. verschwenkbare Anordnung des Halteelements wird bewirkt, dass der Stopper (d.h. das Stopperelement) verschieblich bzw. verschwenkbar ist.

Die verschiebliche bzw. verschwenkbare Anordnung des Stoppers bzw. des Halteelements ergibt sich insbesondere daraus, dass der Stopper bzw. das Halteelement über ein zweites Gelenkt, welches z.B. als Scharnier, insbesondere als Filmscharnier ausgebildet ist, mit der ersten Halbschale verbunden ist. Das zweite Gelenkt (insbesondere als Scharnier oder Filmscharnier) ergibt sich insbesondere dadurch, dass eine Biegerille auf der der Innenfläche der ersten Halbschale abweisenden Halteelementseite ausgebildet ist.

Bevorzugt ist, dass am längsseitigen Rand der zweiten Halbschale eine Halteelementauflage, insbesondere mit Einrastrille, ausgebildet ist.

Die Vorrichtung ist insbesondere derart ausgestaltet, dass beim Schliessen der beiden Halbschalen das Halteelement über den längsseitigen Rand der zweiten Halbschale aus der sich schliessenden Kammer hinaus rutscht.

Zur besseren Bedienung der Vorrichtung, sind an beiden Halbschalen, insbesondere am jeweiligen längsseitigen Rand der Halbschalen, Flügel angeordnet. An den Flügeln kann ein Verschluss, insbesondere ein Schnappverschluss, ausgebildet sein.

In geschlossener Stellung ist das Halteelement (im Wesentlichen) ausserhalb der umschlossenen Kammer positioniert und liegt vorzugsweise zwischen den Flügeln.

Das Halteelement ist insbesondere selbsttragend ausgeführt.

Optional können mehrere Halteelemente, insbesondere z.B. zwei Arme, vorliegen, wobei jedes Halteelement bzw. jeder Arm mindestens einen Stopper trägt, das heisst, zum Beispiel einen ersten Stopper an einem ersten, vorderen Arm zur Verhinderung eines Abgleitens der Linse oder deren Haptik aus der offene Kammer nach vorne und einen zweiten Stopper an einem zweiten, hinteren Arm zur Verhinderung eines Abgleitens der Linse oder deren Haptik aus der offene Kammer nach hinten, insbesondere einen ersten Stopper für die erste oder vordere Haptik der Linse und einen zweiten Stopper für die zweite oder hintere Haptik der Linse.

Vorteilhafterweise ist das Halteelement integraler Teil der Vorrichtung und/oder besteht aus Kunststoff. Die erfinderische Vorrichtung kann in einem einzigen Spritzgussgang in einem Stück hergestellt werden.

Zweckmässig ist, dass jede Halbschale mit einer Auflage, z.B. als Gleitschienen ausgebildet, ausgestattet ist.

Die umschlossene Kammer bildet in der geschlossenen Stellung der Halbschalen einen Kanal, insbesondere einen Ladekanal.

An der Vorrichtung kann an einer der Halbschalen eine Steckvorrichtung zum Einstecken in eine Aufnahmeöffnung eines Injektorgehäuses ausgebildet sein.

Die hier vorgestellte alternative Vorrichtung eignet sich bzw. ist insbesondere ausgebildet als Kartusche zum Einsetzen in einen Injektor, insbesondere in ein Injektorgehäuse. Somit ist weiter offenbart ein Injektor mit einem Injektorgehäuse und einem im Injektorgehäuse längsverschiebbaren Stössel zur Verwendung mit einer als Kartusche ausgebildeten Vorrichtung wie hierin beschrieben.

Weiter offenbart ist hierin ein weiteres Verfahren zum Falten einer intraokularen Linse beinhaltend die Schritte:
- Bereitstellen einer Ladefläche, welche zumindest durch eine erste Halbschale und eine zweite Halbschale definiert ist, und eines die erste und zweite Halbschale überspannendes Halteelement mit Stopper (i.e. Stopperelement), wobei die erste Halbschale auf einer Seite über ein erstes Gelenk mit der zweiten Halbschale gelenkig verbunden ist und auf der gegenüberliegenden Seite über ein zweites Gelenk mit dem Halteelement gelenkig verbunden ist, wobei das Halteelement mit dem längsseitigen Rand der zweiten Halbschale gleitend in Kontakt ist,
- Aufbringen (insbesondere auflegen oder aufschieben) einer Linse auf die Ladefläche, indem der gesamte Linsenkörper der Linse auf die Ladefläche gebracht wird,
- Zusammenführen der beiden Halbschalen über das erste Gelenkt, indem die Seite der ersten Halbschale mit dem Halteelement durch Drehung um das erste Gelenk dem Rand der zweiten Halbschale zugeführt wird, wobei gleichzeitig das Halteelement über den Rand der zweiten Halbschale aus einem sich zwischen den beiden schliessenden Halbschalen bildenden Hohlraum gleitet, was gleichzeitig eine Drehung des Halteelements (und somit des Stoppers) um das zweite Gelenk bedeutet.

Das Verfahren zeichnet sich insbesondere dadurch aus, dass bei der Verwendung einer Linse mit zwei Haptiken die vordere Haptik der Linse, über einen Stopper und die hintere Haptik über einen Stössel zum Linsenkörper hin gestossen wird bzw. werden. Vorteilhafterweise kann die Linse in spannungslosem Zustand auf die Ladefläche aufgebracht bzw. geführt werden. Die Linse liegt zweckmässigerweise auf der Innenfläche der beiden Halbschalen auf. Die Linse ist gegebenenfalls an ihren Rändern durch die beiden Halbschalen eingefasst und wird mit den Halbschalen zusammen, insbesondere in ungefähr gleicher Richtung, gefaltet. Der Verfahrensschritt zum Zusammenführen der beiden Halbschalen beinhaltet insbesondere ein Zusammenführen bis zum gegenseitigen Aneinanderstossen der beiden längsseitigen Ränder der beiden Halbschalen, wobei das Halteelement eingeklemmt wird.

Zusätzliche Vorteile und Ziele der vorliegenden Erfindung ergeben sich aus der nachfolgenden Beschreibung.

### KURZBESCHREIBUNG DER FIGUREN

Weitere bevorzugte Ausführungen der Erfindung ergeben sich aus der nun folgenden Beschreibung anhand der Figuren. Es zeigen schematisch, in nicht massstabsgetreuen Darstellung:
- Figur 1:: eine schräge Ansicht der erfindungsgemässen Vorrichtung in offener Stellung mit aufgenommener intraokularer Linse;
- Figur 2a:: eine Vorderansicht der erfindungsgemässen Vorrichtung in offener Stellung;
- Figur 2b:: ein Ausschnitt aus Figur 2a die Auflageverbindung zwischen dem freien Ende eines Deckelglieds und dem Rand einer zweiten Halbschale zeigend;
- Figur 3:: eine Vorderansicht der erfindungsgemässen Vorrichtung in geschlossener Stellung;
- Figur4:: eine Vorderansicht der erfindungsgemässen Vorrichtung in geschlossener Stellung mit aufgenommener intraokularer Linse;
- Figur 5a:: eine optische Linse mit eingefalteter erster und zweiter bzw. vorderer und hinterer Haptik;
- Figur 5b:: eine optische Linse mit einer ersten eingefalteten Haptik und einer zweiten geöffneten Haptik;
- Figur 6:: eine schräge Ansicht eines Injektors mit eingesteckter erfindungsgemässer Vorrichtung in offener Stellung;
- Figur 7:: eine weitere schräge Ansicht eines Injektors mit eingesteckter erfindungsgemässer Vorrichtung in offener Stellung;
- Figur 8:: eine weitere schräge Ansicht eines Injektors mit eingesteckter erfindungsgemässer Vorrichtung in offener Stellung;
- Figur 9:: eine schräge Ansicht eines Injektors mit eingesteckter erfindungsgemässer Vorrichtung in geschlossener Stellung;
- Figur 10:: eine erfindungsgemässe Vorrichtung mit alternativer Deckelgliedausführung in offener Stellung: (a) eine Ansicht schräg von vorne, (b) eine Ansicht schräg von hinten.

### DETAILIERTE BESCHREIBUNG DER FIGUREN

Im Folgenden stehen gleiche Bezugsziffern für gleiche oder funktionsgleiche Elemente (in unterschiedlichen Figuren). Ein zusätzlicher Apostroph kann zur Unterscheidung mehrerer gleicher, gleichartiger, funktionsgleicher oder funktionsähnlicher Elemente dienen.

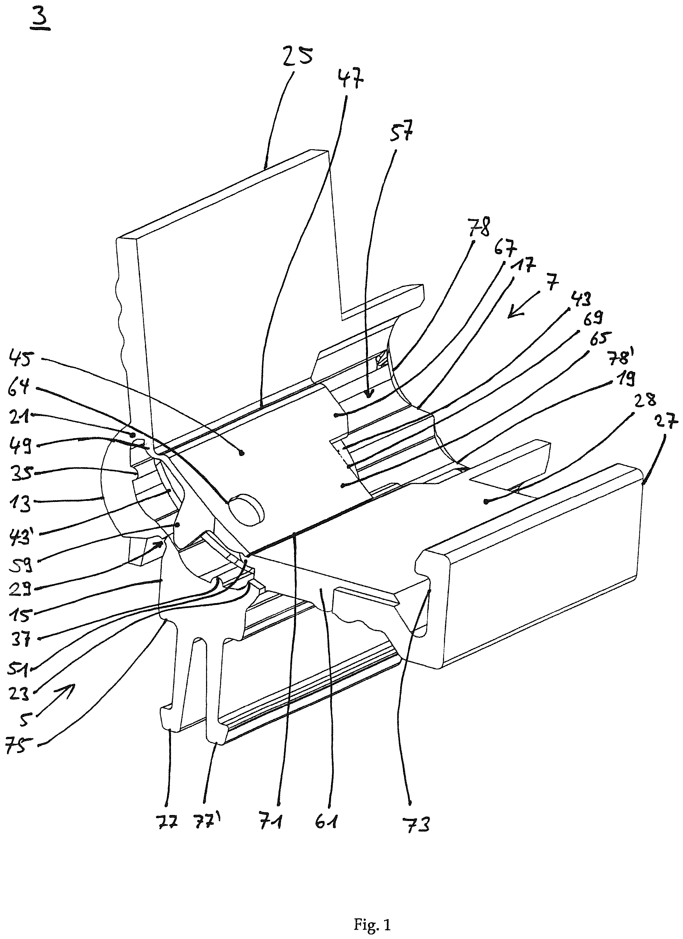

In den Fig. 1-4 ist eine erfindungsgemässe Vorrichtung in Form einer in ein Injektorgehäuse 1 einsetzbaren Kartusche 3 dargestellt. Alternativ kann die erfindungsgemässe Vorrichtung Teil eines Injektors sein bzw. fix im Injektor integriert bzw. ausgebildet sein.

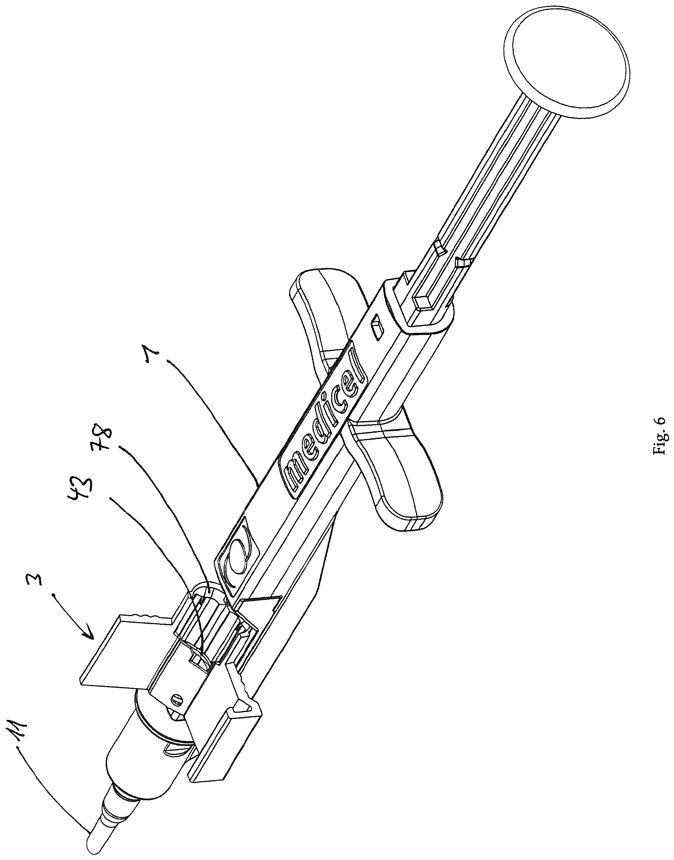

In Fig. 6-9 ist ein Injektor mit eingesetzter Kartusche 3 dargestellt.

Der Injektor ist ein Operationswerkzeug mit einem hülsenartigen Gehäuse 1 und einem im Gehäuse axial beweglich aufgenommenen Stössel 9. Vorzugsweise ist dem Stössel 9 ein elastischer Stempel aufgesetzt. Im Mantel des Gehäuses ist vorzugsweise eine Ausnehmung, z.B. Schlitz, vorgesehen, in welche ein Linsenträger, d.h. insbesondere die hierin beschriebene Kartusche 3, ladbar ist. Der Linsenträger bzw. die Kartusche 3 besitzt einen vorzugsweise zylindrischen Ladekanal 39, an welchen sich axial eine sich zur Spitze hin verjüngende Injektordüse 11 (distales Ende des Linsenträgers) anschliesst. Der Linsenträger bzw. die Kartusche 3 wird derart im Injektorgehäuse 1 eingelegt bzw. gehalten, dass der Stössel 9 fluchtend mit dem Ladekanal ist. Beim Vorschub dringt der Stössel 9 in den Ladekanal 39 ein und schiebt die Linse aus der Injektordüse 11.

Die Kartusche 3 hat eine vorderes Ende 5 und ein hinteres Ende 7. In ein Injektorgehäuse 1 eingesetzt kann vom hinteren Ende 7 her der Stössel 9 in und durch die Kartusche 3 Richtung vorderes Ende 5 der Kartusche 3 und weiter in eine Injektionskanüle 11 gestossen werden.

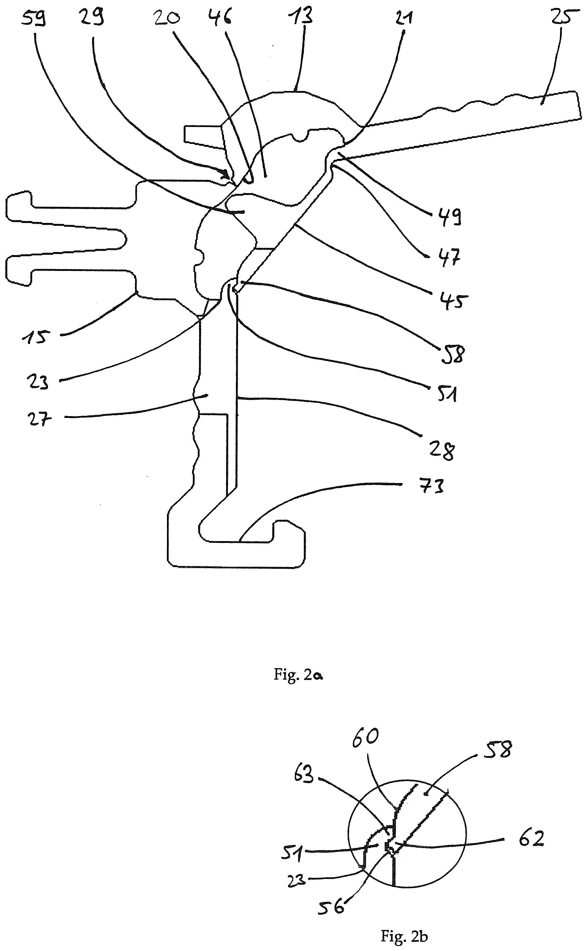

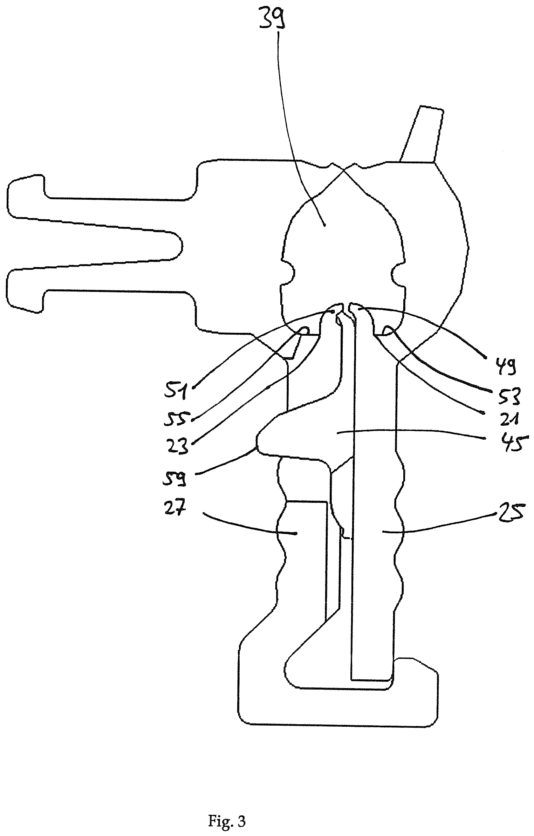

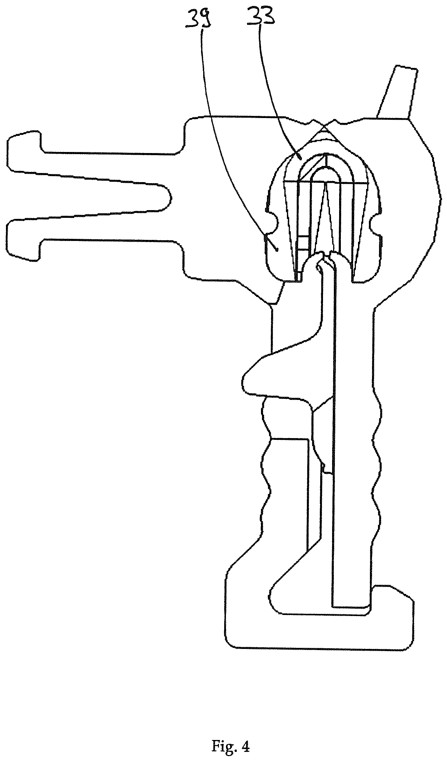

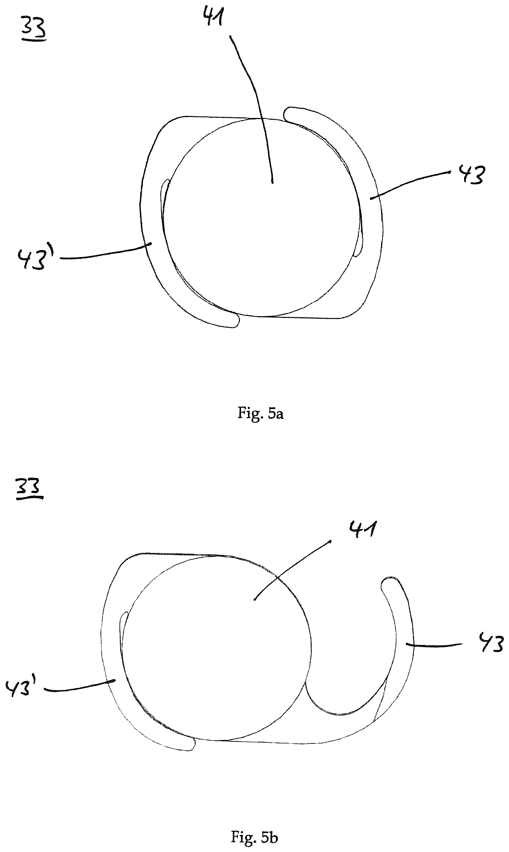

Die Kartusche 3 beinhaltet zwei Halbschalen 13 und 15, welche über ein erstes Gelenk 29 miteinander gelenkig verbunden sind. Die zwei Halbschalen 13 und 15 sind insbesondere zylindersegmentartig ausgeführt und längsverlaufend gelenkig miteinander verbunden. Die zwei gelenkig miteinander verbundenen Halbschalen 13 und 15 bilden zusammen eine Doppelhalbschale. Jede Halbschale 13, 15 weist innenseitig einen offenen Halbkanal mit einer Innenfläche 17 bzw. 19 auf. Die Innenflächen 17 und 19 bilden zusammen eine Ladefläche 20. Die Ladefläche 20 wird längsseitig von einem ersten Rand 21 und einem zweiten Rand 23 begrenzt. Anders ausgedrückt: jede Halbschale 13, 15 weist auf einer dem ersten Gelenk 29 abgewandten Seite einen Rand 21 bzw. 23 auf. An jedem Rand 21, 23 ist vorteilhafterweise ein Flügel 25, 27 angeordnet. Die Halbschalen 13 und 15 sind über das erste Gelenk 29, welches zweckmässigerweise z.B. als Scharnier bzw. Filmscharnier ausgeführt ist, in Längsrichtung gelenkig miteinander verbunden. Die beiden Halbschalen 13 und 15 werden dadurch derart aneinandergereiht, dass die Innenflächen 17 und 19 der beiden Halbschalen 13 und 15 nebeneinander liegen, insbesondere in Längsausrichtung aneinandergrenzen (bzw. über das erste Gelenk 29 ineinander übergehen) und eine gemeinsame Ladefläche 20 bilden. Längs oder längsseitig bedeutet hierbei in Ausrichtung entlang der Erstreckung oder Ausrichtung der Halbkanäle. Die Kartusche 3 kann aufgrund des Gelenks 29 von einer offenen Stellung in eine geschlossene Stellung übergeführt werden. In offener Stellung (Fig. 1 und 2) bilden die beiden Halbschalen 13 und 15 eine Art Ladefläche 20, auf welche eine intraokulare Linse 33 aufgelegt werden kann und auf welcher die Linse 33 gegebenenfalls spannungslos gelagert werden kann. Die Linse 33 liegt dabei zwischen den längsseitigen Rändern 21, 23 vorzugweise auf einer Leitstruktur auf, welche hier zum Beispiel aus Gleitschienen 35, 37 besteht. Beim Schliessen nähern sich die längsseitigen Ränder 25 und 27 der beiden Halbschalen 13 und 15 gegenseitig und eine auf der Ladefläche 20 aufliegende künstliche intraokulare Linse 33 wird erfasst und gefaltet. In geschlossener Stellung (Fig. 3 und 4) bilden die beiden Halbschalen 13 und 15 zusammen einen geschlossenen Kanal, i.e. den vorgenannten Ladekanal 39, welcher dazu dient die Linse 33 in gefaltetem Zustand zur Injektion in ein Auge bereit zu halten. Intraokulare Linsen 33 bestehen im Wesentlichen aus einem optischen Linsenkörper 41 und einer oder mehreren Haptiken 43, vorzugsweise einer ersten und einer zweiten Haptik 43, 43' (Fig. 5), welche für gewöhnlich in der Linsenebene von der Peripherie des Linsenkörpers 41 spiralartig (insbesondere gleichgerichtet spiralartig) abstehen und federnd ausgebildet sind. Um ein Einklemmen der Linse 33 und insbesondere ihrer Haptik 43 beim Schliessen der Halbschalen zu vermeiden, ist die Kartusche 3 mit einem Deckelglied 45 ausgestattet. Das Deckelglied 45 ist am längsseitigen Rand 21 der ersten Halbschale 13 beweglich insbesondere verschieb- oder klappbar (ähnlich einer einflügeligen Schwingtür), angeordnet bzw. befestigt. Das Deckelglied 45 ist vorteilhafterweise als Deckelplatte ausgeführt, insbesondere als plane, formstabile Deckelplatte. In einer offenen Stellung der Kartusche 3 überspannt das Deckelglied 45 die Ladefläche 20 vom längsseitigen Rand 21 der ersten Halbschale 13 bis zum längsseitigen Rand 23 der zweiten Halbschale 15. Das Deckelglied 45 ist am längsseitigen Rand 21 der ersten Halbschale 13 vorteilhafterweise über ein zweites Gelenk 47 beweglich fixiert. Zweckmässigerweise ist das Gelenk 47 ein Scharnier, insbesondere ein Filmscharnier, und ist als Biegerille bzw. Falzbereich ausgestaltet.

Die Drehachsen des ersten und zweiten Gelenks 29 und 47 sind vorzugsweise parallel zueinander ausgerichtet.

Das Deckelglied 45 schliesst zum längsseitigen Rand 23 hin aufgrund seiner Schwerkraft und/oder einer Federkraft im zweiten Gelenk 47. Da das Deckelglied 45 selbsttragend (d.h. genügend steif) ist, bildet sich in offener Stellung der Kartusche 3 eine gedeckte Kammer 46 zwischen erster Halbschale 13, zweiter Halbschale 15 und Deckelglied 45. Das zweite Gelenk 47 ist vorzugsweise an einer vom längsseitigen Rand 21 der ersten Halbschale 13 abstehenden Leiste 49 angeordnet. Auf der Gegenseite, d.h. am längsseitigen Rand 23 der zweiten Halbschale 15 ist vorzugsweise eine zweite abstehende Leiste 51 angeformt, welche bei offener Kartusche 3 als Auflage für das Deckelglied 45 dient. Die Leisten 49 und 51 sind beide zweckmässigerweise gekrümmt und gegebenenfalls mit sich verjüngenden Enden bzw. Längsseiten ausgeführt. Das sich vorzugsweise verjüngende Ende bzw. die sich vorzugsweise verjüngende Längsseite der Leiste 49 geht zweckmässigerweise in ein Filmscharnier über. Die Krümmung der Leisten 49, 51 ist insbesondere eine Wölbung über deren Länge, die Krümmung der Leisten 49, 51 ist dergestalt, dass sich die beiden Leisten bei geschlossener Kartusche zu einer halbkreisartigen Ausbuchtung ergänzen, welche sich in den geschlossenen Kanal 39 erstreckt. Die Leisten 49, 51 bilden in seitlicher Fortführung der Innenflächen 17, 19 am jeweiligen längsseitigen Rand 21, 23 eine Gegenfläche zur jeweiligen Innenfläche 17, 19, wodurch beidseitig eine Art innenseitige Nut 53, 55 am jeweiligen längsseitigen Rand 21, 23 entsteht (Fig. 3). Nut 53 bzw. 55 und Gleitschienen 35 bzw. 37 können als Leitsystem zur Einführung einer Linse 33 zusammenwirken. Die Leisten 49, 51 können in Längsrichtung durchgehend (wie in den Figuren gezeigt) oder unterbrochen (nicht bildlich gezeigt) ausgebildet sein. Das Leitsystem ist zweckmässigerweise parallel zur Längserstreckung der Halbschalen 13,15 ausgerichtet.

Die Leiste 49 ist kammeraussenseitig vorzugsweise mit einer konkaven Biegerille ausgebildet. Die Biegerille weist insbesondere eine linienförmige Stoffverdrängung in Längsrichtung der ersten Halbschale 13 auf, wodurch eine Biegefähigkeit des Materials erzeugt wird. Dadurch kann das Deckelglied 45 gelenkig zum ersten Flügel 25 hin geklappt werden. Die Biegerille funktioniert insbesondere als Filmscharnier.

Die Leiste 51 ist kammeraussenseitig vorzugsweise mit einer konkaven Rille 56 versehen. In dieser Rille 56 kann bei entsprechender Ausgestaltung des freien Endes 58 des Deckelglieds 45 das Deckelglied 45 bzw. dessen freies Ende 58 einrasten. Rille 56 und freies Ende 58 sind derart ausgestaltet, dass beim Zudrücken (d.h. bei Betätigung per Hand) der beiden Flügel 25, 27 das Deckelglied 45 bzw. dessen freies Ende 58 aus der Rille 56 gestossen wird um der Flügelfläche 28 entlang zu gleiten. Das freie Ende 58 des Deckelglieds 45 ist hierfür zweckmässigerweise kammerinnenseitig mit einer sich verjüngenden konvexen Rundung 60 ausgestaltet, welche vorzugsweise mit einem wulstartigen Saum 62 endet. Die Leiste 51 bildet somit eine Art Deckelgliedauflage 63 mit Einrastrille 56.

Wenn das Deckelglied 45 gegenüber den Halbschalen, bzw. dessen Innenflächen 17, 19 in Längsrichtung verkürzt ausgebildet ist, kann der freie bzw. ungedeckte Teil 57 der Ladefläche 20 als Ablageplatz für die Linse 33 dienen, auf welchem die Linse, z.B. von Hand, abgelegt werden kann, bevor diese der Ladefläche 20 entlang unter das Deckelglied 45 geschoben wird.

Damit eine vom Ablageplatz 57 her in die gedeckte Kammer 46 eingeschobene Linse 33 nicht auf der vorderen Stirnseite 5 der Kartusche 3 entweicht, ist das Deckelglied 45 mit einem Stopper 59 ausgestattet. Dieser dient auch dem Anlegen der distalen Haptik an den Optikkörper der Linse. Der Stopper ist vorzugsweise an oder nahe der Kartuschenvorderseite 5, d.h. proximal an der Kartusche, angelegt. Der Stopper 59 kann als Ausstülpung ausgebildet sein, welche bei geöffneter Kartusche (insbesondere wenn das freie Ende des Deckelglieds 45 am zweiten Rand 23 bzw. auf der zweiten Leiste 51 aufliegt) aus der Ebene des Deckelglieds innenflächenseitig (d.h. auf Seite der Innenflächen 17, 19) vorsteht.

Zweckmässigerweise weist der zweite Flügel 27 eine Führung, z.B. eine Schneise oder kartuschenstirnseitig eine Aussparung 61, auf, in bzw. an welcher der Stopper 59 entlang gleiten kann, wenn das Deckelglied 45 beim Schliessen der Kartusche 3 über den zweiten Flügel aus der sich bildenden Kammer 39 geführt wird.

In Längsrichtung der Halbschalen 13, 15 entspricht die Abmessung des Flügels 27 der zweiten Halbschale 15 vorzugsweise ungefähr der Abmessung des Deckelglieds 41 oder überragt diese.

Das Deckelglied 45 weist gegebenenfalls einen Durchbruch 64 auf. Dieser liegt vorzugsweise im vorderen Bereich des Deckelglieds 45 und gegebenenfalls in einem Bereich des Deckelglieds 45, welcher der zweiten Innenfläche 19 gegenüberliegt oder - anders ausgedrückt - welcher an den zweiten Rand 23 anstösst. Der Durchbruch 64 ist in Längsrichtung zweckmässigerweise weniger als ein halber Durchmesser, vorzugsweise weniger als ein Drittel des Durchmessers, eines Linsenkörpers 41 von der vorderen Stirnseite 5 der Kartusche 3 oder vom Stopper 59 beabstandet.

Durch diesen Durchbruch 64 kann Gleitflüssigkeit für die Linse 33 eingebracht werden, insbesondere wird dadurch auch der vordere Teil der Kartusche 3, d.h. vor der Linse 33, und die Injektionsdüse 11 mit Gleitflüssigkeit gefüllt. Die Gleitflüssigkeit kann dadurch z.B. eingefüllt werden, nachdem die Linse schon unter das Deckelglied 45 eingeschoben wurde.

Das Deckelglied 45 ist gegenüber den Halbschalen 13, 15 in Längsrichtung am hinteren Kartuschenteil verkürzt, vorzugsweise verkürzt und gestuft, ausgebildet (Fig. 1 und Fig. 6-8). Insbesondere ist das Deckelglied 45 in einem hinteren Bereich 65 des Deckelglieds 45, welcher vorwiegend der zweiten Innenfläche 19 gegenüberliegt oder - anders ausgedrückt - welcher an den zweiten Rand 23 anstösst, gegenüber den Halbschalen 13, 15 verkürzt. In einem hinteren Bereich 67 des Deckelglieds 45, welcher der ersten Innenfläche 17 gegenüber liegt oder - anders ausgedrückt - welcher an den ersten Rand 21 anstösst, kann das Deckelglied 45 ebenfalls gegenüber den Halbschalen 13, 15 verkürzt sein, dies muss es jedoch nicht. Vorzugsweise ist im genannten Bereich 67 über der ersten Innenfläche 17 das Deckelglied 45 weniger verkürzt als im Bereich 65 über der zweiten Innenfläche 19. Insbesondere ist also der Bereich 65 des Deckelglieds, welcher an das lose Ende des Deckelglieds angrenzt, gegenüber der Länge der Kartusche 3 stärker verkürzt als der Bereich 67, welcher an das zweite Gelenk 47 angrenzt. Aufgrund der Verkürzung kann sich am hinteren Teil des Deckelglieds 45 einseitig eine Aussparung ergeben, in welcher ein Stössel 9 eines Injektors bis zum Anschlag 69 und somit bis auf eine eingelegte Linse 33, ggf. den Linsenkörper 41, eingeschoben werden kann, ohne dass das Deckelglied 45 angehoben oder sonst wie verschoben werden muss. Die Breite des Deckelgliedbereichs 67, ist derart bemessen, dass beim Schliessvorgang der Kartusche 3 der Deckelgliedbereich 67 über den bis zum Anschlag 69 der Aussparung vorgestossenen Stössel 9 hinweg rutscht, ohne dass das freie Ende 71 (insb. der Saum 62) des Deckelglieds 45 dabei den Kontakt mit dem Flügel (insbesondere der Flügelinnenseite) verliert.

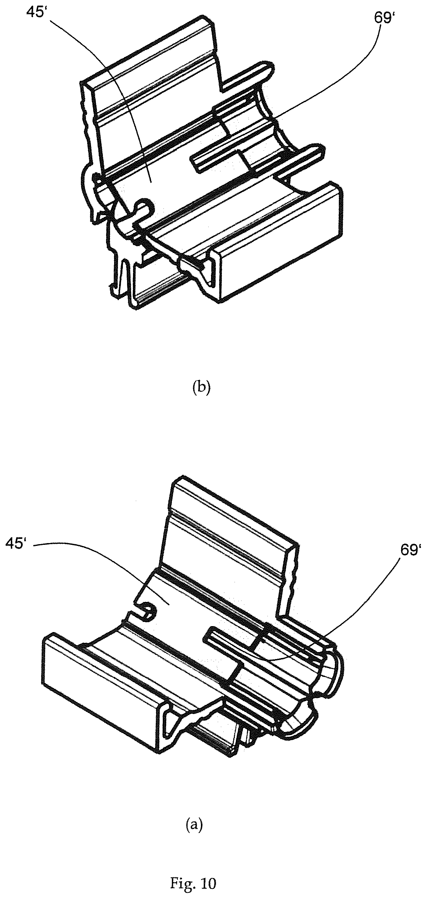

In einer alternativen Ausführung (Fig. 10) hat das Deckelglied 45', welches wie dargestellt gegenüber den Halbschalen 13, 15 in Längsrichtung am hinteren Kartuschenteil verkürzt sein kann, hinten mittig einen Schlitz bzw. eine schlitzartige Aussparung. In diese Aussparung kann ein Stössel eines Injektors bis zum Anschlag 69' und somit bis auf eine eingelegte Linse, ggf. den Linsenkörper, eingeschoben werden, ohne dass das Deckelglied 45' angehoben oder sonst wie verschoben werden muss. Die Aussparung ist zweckmässigerweise ca. 1 bis 3 mm, vorzugsweise 1.5 bis 2.5 mm breit.

In geschlossener Stellung der Kartusche 3 (Fig. 3) liegt das Deckelglied 45 im Wesentlichen ausserhalb der aus der Kammer 46 gebildeten umschlossenen Kammer 39 und vorzugsweise zwischen den Flügeln 25, 27. An den Flügeln 25, 27 ist ein Verschluss 73, insbesondere ein Schnappverschluss, ausgebildet.

An der Halbschale 15 ist eine Steckvorrichtung 75 ausgebildet. Diese Steckvorrichtung 75 dient zum Einstecken in eine Öffnung eines Injektorgehäuses 1. Als Blockiermittel dienen beispielsweise Federbeine 77, 77' mit Widerhaken. Dadurch wird nach dem Einstecken der Kartusche 3 in das Injektorgehäuse 1 eine feste Verbindung zwischen diesen Teilen geschaffen.

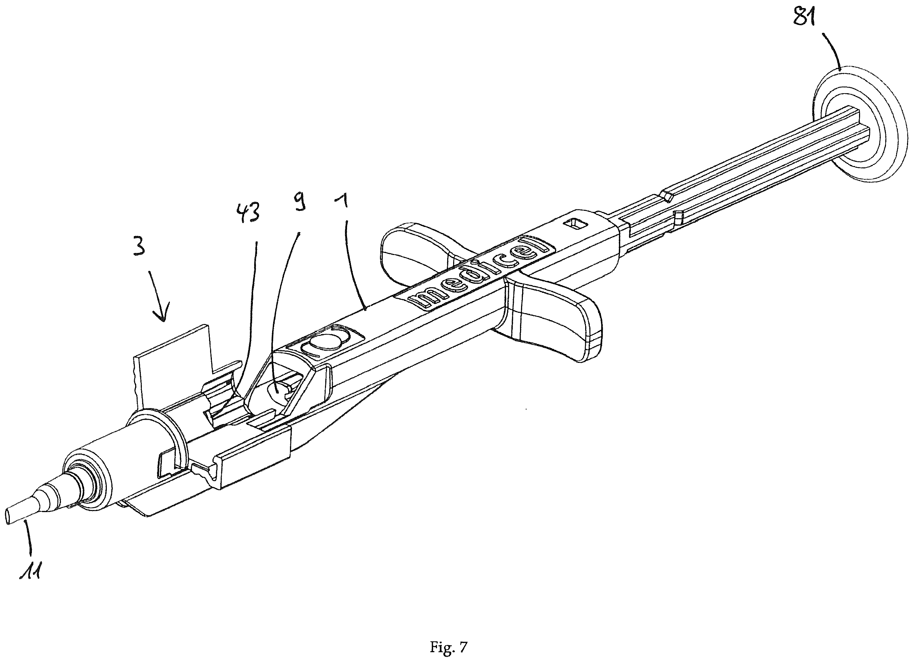

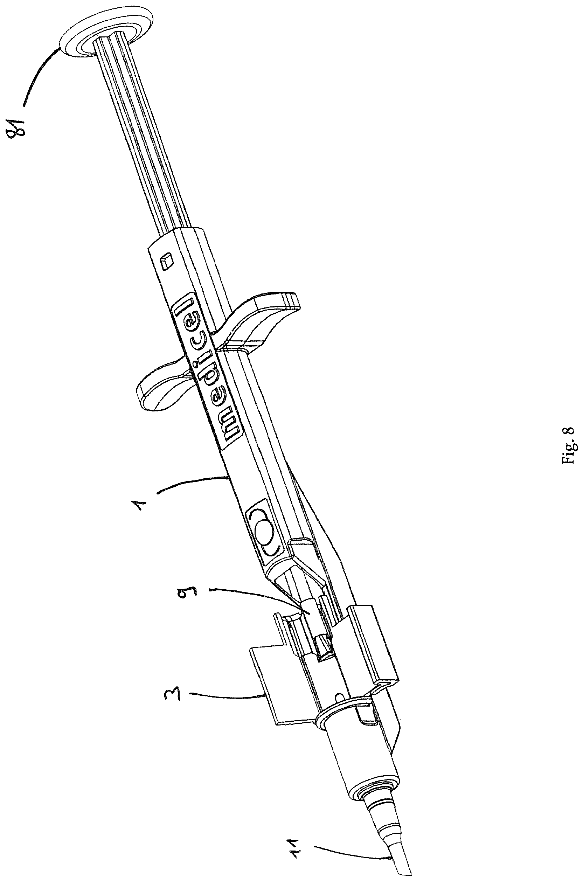

Der in den Figuren 6 bis 9 dargestellte Injektor besteht im Wesentlichen aus dem Injektorgehäuse 1 und dem in diesem verschiebbaren Stössel 9 zum Transportieren und Ausstossen der Linse 33. Bei der gezeigten Ausführungsform stellen die Kartusche 3 und ggf. die Injektordüse 11 separate in das Injektorgehäuse 1 einsetzbare Teile dar, sie können jedoch auch als ein Teil ausgebildet sein. Fig. 6 und Fig. 7 zeigen einen Injektor mit im Injektorgehäuse 1 eingesetzter und mit einer Linse 33 besetzter Kartusche 3 (es ist lediglich die hintere Haptik 43 der Linse 33 zu sehen). Der Stössel 9 ist in einer Ausgangsstellung im Injektorgehäuse gelagert (in Fig. 6 nicht sichtbar), vorzugsweise arretiert, sodass er beim Einsetzen der Kartusche 3 nicht im Weg ist. Am hinteren Ende 7 (proximal) der Kartusche 3, d.h. der Halbschalen 13,15, kann optional zu den Innenflächen 17, 19 hin umlaufend eine Schräge 78, 78' ausgebildet sein, welche der Einführung eines Stössels 9 dienen kann. In Fig. 8 ist der Stössel 9 im Injektor in teilvorgeschobener Stellung zu sehen. In dieser teilvorgeschobenen Stellung reicht der Stössel 9 bis an den hinteren Rand bzw. Anschlag 69 des Deckelglieds 45. Bei dieser Stellung des Stössels 9 ist die Linse 33 mit vorderer und hinterer Haptik 43', 43 gegebenenfalls in sich zusammengestossen. Linse 33 und Haptiken 43, 43' sind dabei unter dem Deckelglied 45 in der Kammer 46 positioniert; d.h. die Haptiken stehen nirgends vor. Eine axiale Verschiebung der Linse 33 wird einerseits durch den Stopper 59 und andererseits durch den Stössel 9 verhindert. Der Stössel 9 kann gegebenenfalls im Injektorgehäuse 1 arretiert sein. Es ist somit möglich, bereits in der Fabrik den Injektor mit der einzusetzenden Linse 33 zu versehen. Dies kann z.B. bei trocken zu lagernden Linsen eine Option sein. Vor der Operation ist somit lediglich die Kartusche durch den Operateur oder dessen Assistenz zu schliessen, indem der erste Flügel 25 gegen den zweiten Flügel 27 bewegt wird, um die Linse 33 dabei zu falten und das Deckelglied 45 aus dem Kanal 46 (bzw. aus dem sich bildenden Kanal 39) zu schieben.

In einer weiteren Ausführungsform (in den Figuren nicht gezeigt) hat die Kartusche Stopper an zwei Seiten des Deckelglieds 45 (insbesondere an oder nahe der vorderen Stirnseite und an oder nahe der hinteren Stirnseite der Kartusche). Die Stopper befinden sich dabei vorzugsweise am Deckelglied selbst, wobei bezüglich der Ausrichtung der Kartusche zweckdienlicherweise ein Stopper an der Vorderkante und ein Stopper an der Hinterkante des Deckelglieds angeordnet ist. Dies ermöglicht z.B. das Lagern der Linse in der Kartusche (i.e. vorgeladene Linse). Eine derart vorgeladene Linse kann gegebenenfalls in einem mit steriler Flüssigkeit gefüllten Behälter aufbewahrt werden, ohne dass dabei die Linse aus der Kartusche herausfallen kann. Nach Öffnen des Behälters wird die Kartusche aus diesem Behälter entnommen und in das Injektorgehäuse 1 eingesetzt. Auch hier kann nun die Kartusche (wie oben beschrieben) geschlossen werden, wobei das Deckelglied 45 mit den beiden Stopper seitlich aus dem Kanal 46 (bzw. aus dem sich bildenden Kanal 39) geschoben wird.

Ist bei teilvorgestossenem Stössel 9 die Linse 33 mit Linsenkörper 41 und Haptiken 43, 43' unter dem Deckelglied 45 positioniert, kann die Kartusche 3 vorteilhafterweise geschlossen werden, ohne dass der Linsenkörper 41 oder die Haptiken 43, 43'während des Schliessvorgangs zwischen den Rändern 21 und 23 bzw. zwischen den Flügeln 25 und 27 der Kartusche eingeklemmt werden. Der Schliessvorgang der Kartusche läuft insbesondere wie folgt ab: Vorzugsweise per Hand werden die Flügel 25, 27 der Kartusche 3 zusammengeführt. Dabei rutscht das Deckelglied 45 von seiner Raststellung am Rand 23, insbesondere aus der Rille 56 auf die innenseitige Flügelfläche 28 des zweiten Flügels 27 und dieser Flügelfläche 28 entlang aus dem sich schliessenden Hohlraum 46 bzw. aus dem sich bildenden Hohlraum 39 heraus.

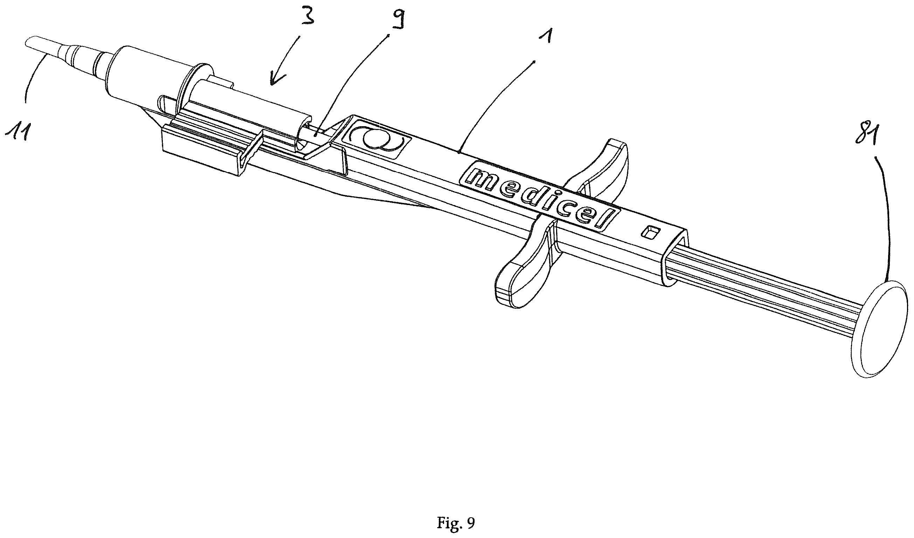

In Fig. 9 ist der Injektor mit geschlossener Kartusche 3 und teileingeschobenem Stössel 9 zu sehen. Dass der Stössel 9 nur teilweise in die Kartusche 3 eingeschoben ist, ist daran zu erkennen, dass das Stösselende 81 (gegenüber Fig. 8) mit unveränderter Länge aus dem Injektorgehäuse 1 vorsteht. In dieser Stellung ist die Linse 33 bereit zur Injektion. Durch Vorstossen des Stössels 9 wird die Linse 33 durch die Kanüle (Injektordüse) 11 ausgestossen, um dabei z.B. in ein Auge injiziert zu werden.

In einer weiteren Ausführungsform (in den Figuren nicht gezeigt) ist das plattenförmige Deckelglied 45 durch zumindest einen Arm, insbesondere durch einen Arm oder zwei Arme ersetzt, wobei der Arm bzw. jeder der Arme bei offener Stellung der Kartusche 3 die Ladefläche 20 vom längsseitigen Rand 21 der ersten Halbschale 13 bis zum längsseitigen Rand 23 der zweiten Halbschale 15 überspannt. Stopper, welche am Arm bzw. an jedem der Arme angeformt sind, dienen (wie oben mit Bezug auf das Deckelglied 45 beschrieben) der Positionssicherung der Linse 33, insbesondere auch deren Haptik. Der oder die Arme sind insbesondere derart ausgeführt, dass der oder die Stopper 59, wie oben beschrieben und in den Figuren dargestellt, angeordnet sind. Insbesondere ist der Stopper vorzugsweise als Ausstülpung des Arms ausgebildet, welche bei geöffneter Kartusche (insbesondere wenn das freie Ende des Arms am zweiten Rand 23 bzw. auf der zweiten Leiste 51 aufliegt) vom Arm aus halbschaleninnenflächenseitig (d.h. auf Seite der Innenflächen 17, 19 der Halbschalen 13, 15 und somit auf die Innenflächen 17, 19 der Halbschalen 13, 15 weisend) vorsteht. Fig. 2a und Fig. 2b zeigen insbesondere eine Vorderansicht der erfindungsgemässen Vorrichtung, wobei diese Vorderansicht gleichermassen eine Vorrichtung mit Halteelement 45 ausgeführt als plattenförmiges Deckelglied oder ausgeführt als Arm darstellt. In jedem Fall ist zweckmässig, dass der Stopper 59 beim Übergang in die geschlossene Stellung der Kammern 13 und 15 aus der sich bildenden Ausstosspassage (sowie deren Verlängerung) weicht bzw. gleitet. Im in Fig. 2a gezeigten Beispiel weicht der Stopper insbesondere aufgrund seiner kartuschenendständigen Position, welche es dem Stopper erlaubt sich beim Übergang in die geschlossene Stellung der Kammern 13 und 15 an der vorderen Stirnseite des zweiten längsseitigen Rands 23 und des Flügels 27 der zweiten Halbschale 15 entlang in der Aussparung 61 zu bewegen. Zweckmässigerweise ist die Stirnseite des zweiten längsseitigen Rands 23 und des Flügels 27 der zweiten Halbschale 15 zurückversetzt aufgrund dessen sich die stirnseitige Aussparung 61 ergibt. Bei nicht kartuschenendständiger Position eines Stoppers könnte eine Aussparung als Schneise in Rand 23 und Flügel 27 in orthogonaler Richtung bezüglich der Drehachsen des ersten und zweiten Gelenks 29 und 47 ausgebildet sein.

Das Deckelglied, insbesondere der Arm oder das plattenförmige Deckelglied 45 können allgemein als Halteelemente für den Stopper bezeichnet werden.

### Zusammenfassend kann folgendes festgehalten werden:

Eine Vorrichtung zum Falten einer intraokularen Linse 33 weist zwei durch ein Scharnier 29 verbundene Halbschalen 13, 15 auf, welche z.B. mittels an den Halbschalen 13, 15 ausgebildeten Flügeln 25, 27 relativ zueinander bewegt und gegeneinander geschlossen werden können, um eine eingelegte intraokulare Linse 33 zu falten und gleichzeitig in einem Ladeträgerkanal, welcher sich durch das Schliessen der Halbschalen 13, 15 bildet, injizierbereit zu halten. Im weiteren weist die Vorrichtung zum Falten vorzugsweise ein Halteelement (insbesondere ausgeführt als ein Arm (in den Figuren nicht explizit gezeigt) oder ein Deckelglied 45) auf, welches in einer offenen Stellung der Halbschalen 13, 15 von einem ersten längsseitigen Rand 21 an der ersten Halbschale 13 zu einem zweiten längsseitigen Rand 23 an der zweiten Halbschale 15 eine Brücke über die Innenflächen 17, 19 der Halbschalen 13 und 15 bildet. Ein Verklemmen der Linse 33, insbesondere des Linsenkörpers 41 oder der Haptiken 43, 43', lässt sich dadurch verhindern, dass das Halteelement (insbesondere wenn es als Deckelglied 45 ausgeführt ist) vor und während dem Falten über der Linse liegt, die Linse auf den Halbschalen sichert und dadurch die Linse daran hindert (insbesondere während dem Falten, d.h. beim Schliessen der beiden Halbschalen) in den Zwischenraum zwischen den Rändern 21 und 23 und/oder zwischen den Flügeln 25 und 27 zu dringen. Unterstützend wirkt hierbei die nutartige Ausgestaltung (Nut 53, 55) der Halbschalen 13, 15 an den beiden längsseitigen Rändern 21, 23, welche die Linse teilweise einrahmen. Beim Schliessen der beiden Halbschalen 13, 15 rutscht das Halteelement (insbesondere das Deckelglied 45 oder der Arm), sowie gegebenenfalls der oder die Stopper 59 aus dem sich bildenden Ladekanal (i.e. Hohlraum 36) hinaus bzw. von dem sich bildenden Ladekanal weg, um den spätere Ausstoss der gefalteten Linse bzw. deren Injektion in ein Auge nicht zu behindern.

Nachfolgend wird der Gegenstand anhand von Beispielen erläutert.

### ANWENDUNGSBEISPIEL 1

### Anwendungsfall und Problemstellung:

Aufgrund des einfachen, wenig fehlerbehafteten Ladens einer Linse von hinten in eine in sich geschlossene Injektionskartusche wird diese von hinten her zu ladende Art der Injektionskartusche von vielen Anwendern bevorzugt. Bei bekannten, von hinten geladenen Injektionskartuschen müssen jedoch starre Kolbenenden verwendet werden, um die Linse in das Auge injizieren zu können. Das Problem starrer Kolbenenden ist, dass diese die Linse beschädigen können, insbesondere wenn eine Linse durch eine möglichst kleine Inzisionen in ein zu operierendes Auge transplantiert werden soll, da, umso dünner das Kolbenende ist, die Gefahr steigt, dass das Kolbenende gegebenenfalls fehlerhafterweise in die Linse eindringen kann oder diese zumindest verbiegt oder verkratzt.

Bei Verwendung einer von hinten bestückten Injektionskartusche kann die Intraokularlinse nicht vorgefaltet werden, weshalb die Linse durch die Innengeometrie der Injektionsdüse beim Vorschieben durch den Stempel gefaltet werden muss. Aufgrund der sich vom Linsenquerschnitt bis auf den Inzisionsdurchmesser verjüngenden Geometrie kann kein Kolben (hierin auch Stössel genannt) mit flexiblem und der Form anpassendem Ende (Stempel) verwendet werden. Ein flexibler Stempel müsste nämlich einen Formschluss mit der Kartusche bilden, andernfalls bestünde ein sehr hohes Risiko, dass bei Vorschub des Stempels die hintere Linsenhaptik zwischen Stempel und Kartuschenwand eingeklemmt wird. Das Volumen eines solchen flexiblen Stempels wäre jedoch so gross, dass sich dieser beim Vorschieben des Kolbens stark komprimieren müsste und es in Folge dessen zu einer Penetration des Stempels durch die Kolbenspitze sowie zur Deformation und einem Abscheren des Stempels kommen kann. Die notwendigen Kräfte für das Zusammenpressen des Stempels würden ausserdem die Injektionskraft für den Anwender auf ein unakzeptables Mass steigen lassen.

### Problemlösung:

Eine Ladekammer wird mit integriertem Linsenhalter mit Linsenabdeckung (Deckelglied) als Teil eines Injektionssystems verwendet.

Die Ladekammer ist im beschriebenen Anwendungsfall, ein von hinten geladenes System, nach hinten offen und ermöglicht dem Anwender ein Bestücken der Ladekammer mit der Intraokularlinse von hinten. Die Linsenabdeckung fungiert dabei als Einführhilfe für die Linse. Nach Einführen der Linse und der Anwendung eines Viskoelastikums als Gleithilfe wird die Ladekammer geschlossen, die Linse dabei vorgefaltet und die Linsenabdeckung durch einen Scharniermechanismus zwischen den zwei Flügeln der Ladekammer selbsttätig eingefaltet. Durch dieses Vorfalten wird der Querschnitt gegenüber einer dem aktuellen Stand der Technik entsprechenden von hinten geladenen Injektionskartusche signifikant reduziert und somit kann ein formschlüssiger, die Linse nicht verletzender flexibler Stempel verwendet werden. Dieser flexible Stempel wird im Anschluss an den Faltvorgang vorgeschoben, wobei die Linse somit in das Auge injiziert werden kann.

### ANWENDUNGSBEISPEIL 2

### Anwendungsfall und Problemstellung:

Bei einem vorgeladenen System muss die Intraokularlinse während des Transports und der Lagerung vor einem Verrutschen und Herausgleiten aus der Injektionskartusche beziehungsweise der Ladekammer geschützt werden. Dies wird durch einen Linsenhalter realisiert. Ein handelsüblicher Linsenhalter hat jedoch den Nachteil, dass der Anwender diesen selber entfernen muss. Bei einem unvorsichtigen Entfernen kann es zu einer Dislokation der Linse kommen. Da der Anwender bei einem vorgeladenen System keine Manipulation an der Linse vornimmt beziehungsweise vornehmen darf, kann es im darauf folgenden Schliessvorgang der Ladekammer beziehungsweise beim anschliessenden Vorschub des Stempels zm Einklemmen der Linse beziehungsweise deren Haptiken führen. Zudem stellt das Entfernen des Linsenhalters einen zusätzlichen Schritt für den Anwender dar. Um das System für den Anwender so einfach und komfortabel wie möglich zu gestalten, sollte dieser zusätzliche Schritt der Entfernung des Linsenhalters nach Möglichkeit entfallen.

### Problemlösung:

Eine Ladekammer wird mit integriertem Linsenhalter mit Linsenabdeckung (Deckelglied) als Teil eines Injektionssystems verwendet.

Die Ladekammer ist im beschriebenen Anwendungsfall, ein vorgeladenes System, nach hinten, oben und auch nach vorne zur Lagerungs- und Transportsicherung vollständig oder zumindest partiell geschlossen. Bei trocken gelagerten Linsen ist die er-Ladekammer bereits in den Injektor eingesetzt. Bei in Flüssigkeiten gelagerten Linsen ist die Ladekammer in einem mit Flüssigkeit gefüllten Behälter aufbewahrt. Aus diesem wird sie durch den Anwender entnommen und in den Injektor eingesetzt. Bei beiden Linsentypen wird nach Injektion eines Viskoelastikums die Ladekammer geschlossen, die Linse dabei vorgefaltet und die Linsenabdeckung durch einen Scharniermechanismus zwischen den zwei Flügeln der Ladekammer eingefaltet. Im Anschluss daran kann der flexible Stempel vorgeschoben und die Linse in das Auge injiziert werden. Die Integration des Linsenhalters und des-sen selbsttätiges Einfalten bringt den Vorteil mit sich, dass der Anwender sich einen Arbeitsschritt und somit Zeit spart, dadurch auch potentielle Fehler durch das bisher notwendige Entfernen des Linsenhalters ausgeschlossen werden und zudem beim Einfalten die Gefahr einer herausrutschenden und sich zwischen den zwei Flügeln einklemmenden Haptik eliminiert wird.

Während vorstehend spezifische Ausführungsformen beschrieben wurden, ist es offensichtlich, dass unterschiedliche Kombinationen der aufgezeigten Ausführungsmöglichkeiten angewendet werden können, insoweit sich die Ausführungsmöglichkeiten nicht gegenseitig ausschliessen.

Während die Erfindung vorstehend unter Bezugnahme auf spezifische Ausführungsformen beschrieben wurde, ist es offensichtlich, dass Änderungen, Modifikationen, Variationen und Kombinationen ohne vom Erfindungsgedanken abzuweichen gemacht werden können.

### BEZUGSZEICHENLISTE

- 1: Injektorgehäuse
- 3: Kartusche
- 5: vordere Stirnseite (bzw. vorderes Ende) der Kartusche
- 7: hintere Stirnseite (bzw. hinteres Ende) der Kartusche
- 9: Stössel (auch Kolben genannt)
- 11: Injektionskanüle bzw. -düse
- 13: erste Halbschale
- 15: zweite Halbschale
- 17: erste Innenfläche, d.h. Innenfläche der ersten Halbschale
- 19: zweite Innenfläche, d.h. Innenfläche der zweiten Halbschale
- 20: Ladefläche
- 21: längsseitiger Rand der Ladefläche an der ersten Halbschale, d.h. erster längsseitiger Rand
- 23: längsseitiger Rand der Ladefläche an der zweiten Halbschale, d.h. zweiter längsseitiger Rand
- 25: erster Flügel
- 27: zweiter Flügel
- 28: Flügelfläche des zweiten Flügels
- 29: erstes Gelenk, insbesondere Filmscharnier
- 33: intraokulare Linse
- 35: erste Gleitschiene bzw. Linsenauflage
- 37: zweite Gleitschiene bzw. Linsenauflage
- 39: umschlossene Kammer, Ladekanal ausgebildet als geschlossener Kanal
- 41: optischer Linsenkörper
- 43, 43': Haptik
- 45, 45': Deckelglied
- 46: offene Kammer, ggf. mit Deckelglied gedeckt
- 47: zweites Gelenk, insbesondere Filmscharnier
- 49: erste Randleiste, d.h. Randleiste am längsseitigen Rand der ersten Halbschale
- 51: zweite Randleiste, d.h. Randleiste am längsseitigen Rand der zweiten Halbschale
- 53: erste Nut
- 55: zweite Nut
- 56: konkave Rinne
- 57: ungedeckter Teil der Ladefläche (Ablageplatz)
- 58: freies Ende des Deckelglieds
- 59: Stopper bzw. Stopperelement
- 60: konvexe Rundung, insb. (kammerinnenseitig) am freien Ende des Deckelglieds
- 61: Schneise oder Aussparung
- 62: Saum am freien Ende des Deckelglieds
- 63: Deckelgliedauflage
- 64: Durchbruch
- 65: gelenkseitiger (d.h. nahe dem Gelenk 47) hinterer Bereich des Deckelglieds
- 67: gelenkferner (d.h. fern vom Gelenk 47) hinterer Bereich des Deckelglieds
- 69, 69': Anschlag
- 71: freies Ende 71 des Deckelglieds
- 75: Steckvorrichtung
- 77, 77': Federbeine
- 78, 78': Schräge, insb. innenseitige Schräge am proximalen Ende der Kartusche
- 81: Stösselende

## Patentansprüche

1. Vorrichtung zur Aufnahme einer intraokularen Linse (33) mit einer ersten und einer zweiten Halbschale (13,15), welche durch ein erstes Gelenk (29) gelenkig miteinander verbunden sind und relativ zueinander von einer offenen Stellung in eine geschlossene Stellung bewegt werden können, wobei
- die Halbschalen (13, 15) in der offenen Stellung eine offene Kammer (46) bilden, welche zum Einlegen einer Linse dient, und
- die Halbschalen (13, 15) in der geschlossenen Stellung eine umschlossene Kammer (39) bilden, welche eine Ausstosspassage definiert und zum Ausstossen der Linse dient,
**dadurch gekennzeichnet,**
**dass** an der ersten der beiden Halbschalen (13) mindestens ein Stopper (59) verschieblich oder verschwenkbar angeordnet ist, welcher in der offenen Stellung die offene Kammer (46) in Ausstossrichtung begrenzt und in der geschlossenen Stellung im Wesentlichen ausserhalb der umschlossenen Kammer (39) seitlich der Ausstosspassage positioniert ist.

2. Vorrichtung nach Anspruch 1, **dadurch gekennzeichnet, dass** der Stopper an einem Halteelement, z.B. einem Deckelglied (45) oder einem Arm, ausgebildet ist, welches in der offenen Stellung die offene Kammer (46) überbrückt bzw. überspannt und in der geschlossenen Stellung im Wesentlichen ausserhalb der umschlossenen Kammer (39) positioniert ist.

3. Vorrichtung nach Anspruch 2, **dadurch gekennzeichnet, dass** der Stopper dadurch verschieblich oder verschwenkbar angeordnet ist, dass das Halteelement am längsseitigen Rand (21) der ersten Halbschale (13) verschieblich bzw. verschwenkbar angeordnet ist.

4. Vorrichtung nach einem der vorangehenden Ansprüche 2 oder 3, **dadurch gekennzeichnet, dass** das Halteelement über ein zweites Gelenkt (47), welches z.B. als Scharnier, insbesondere als Filmscharnier, ausgebildet ist, mit der ersten Halbschale (13) verbunden ist.

5. Vorrichtung nach einem der vorangehenden Ansprüche 2 bis 4 , **dadurch gekennzeichnet, dass** am längsseitigen Rand (23) der zweiten Halbschale (15) eine Halteelementauflage (63), bevorzugt mit Einrastrille (56), ausgebildet ist.

6. Vorrichtung nach einein der vorangehenden Ansprüche 2 bis 5, **dadurch gekennzeichnet, dass** beim Schliessen der beiden Halbschalen (13, 15) das Halteelement über den längsseitigen Rand (23) der zweiten Halbschale (15) aus der sich schliessenden Kammer (46) hinaus rutscht.

7. Vorrichtung nach einem der vorangehenden Ansprüche 2 bis 6 , **dadurch gekennzeichnet, dass** in geschlossener Stellung der Halbschalen (13, 15) das Halteelement zwischen den Rändern der Halbschalen eingeklemmt ist.

8. Vorrichtung nach einem der vorangehenden Ansprüche 2 bis 7, **dadurch gekennzeichnet, dass** in geschlossener Stellung das Halteelement im Wesentlichen ausserhalb der umschlossenen Kammer (39) und vorzugsweise zwischen Flügeln (25, 27) liegt.

9. Vorrichtung nach einem der vorangehenden Ansprüche 2 bis 8, **dadurch gekennzeichnet, dass** mehrere Halteelemente, insbesondere zwei Arme, vorliegen, wobei jedes Halteelement mindestens einen Stopper (59) trägt, insbesondere einen ersten Stopper für die vordere Haptik der Linse und einen zweiten Stopper für die hintere Haptik der Linse (33).

10. Vorrichtung nach einem der vorangehenden Ansprüche 1 bis 9, **dadurch gekennzeichnet, dass** jede Halbschale (13, 15) mit einer Auflage (35, 37), z.B. als Gleitschienen ausgebildet, ausgestattet ist.

11. Vorrichtung nach einem der vorangehenden Ansprüche 1 bis 10, **dadurch gekennzeichnet, dass** an beiden Halbschalen (13, 15), insbesondere am jeweiligen längsseitigen Rand (21, 23) der Halbschalen (13, 15), Flügel (25, 27) angeordnet sind.

12. Vorrichtung nach dem vorangehenden Anspruch 11, **dadurch gekennzeichnet, dass** an den Flügeln (25,27) ein Verschluss (73), insbesondere ein Schnappverschluss, ausgebildet ist.

13. Vorrichtung nach einem der vorangehenden Ansprüche 1 bis 12, **dadurch gekennzeichnet, dass** an einer der Halbschalen (13, 15) eine Steckvorrichtung (73) zum Einstecken in eine Aufnahmeöffnung eines Injektorgehäuses (1) ausgebildet ist.

14. Vorrichtung nach einem der vorangehenden Ansprüche 1 bis 13, **dadurch gekennzeichnet, dass** diese als Kartusche zum Einsetzen in einen Injektor, insbesondere in ein Injektorgehäuse (1), ausgebildet ist.

15. Injektor mit einem Injektorgehäuse (1) und einem im Injektorgehäuse längsverschiebbaren Stössel (9) mit einer als Kartusche ausgebildeten Vorrichtung nach einem der Ansprüche 1 bis 13.

## Claims

1. A device for receiving an intraocular lens (33), comprising a first and a second half-shell (13, 15) which are connected to each other in an articulated manner by a first joint (29) and which can be moved relative to each other from an open position into a closed position, wherein
- the half-shells (13, 15) form an open chamber (46) in the open position, which is used for the insertion of a lens and
- the half-shells (13, 15) form an enclosed chamber (39) in the closed position, which defines an ejection passage and is used for ejecting the lens,
**characterized in that**,
at least one stopper (59) is displaceably or pivotally arranged on the first of the two half-shells (13), said stopper bounding the open chamber (46) in the open position and being positioned substantially outside of the enclosed chamber (39) laterally to the ejection passage in the closed position.

2. The device according to claim 1, **characterized in that** the stopper is formed on a holding element, for example a covering element (45) or an arm, which bridges or spans the open chamber (46) in the open position and is positioned substantially outside the closed chamber (39) in the closed position.

3. The device according to claim 2, **characterized in that** the stopper is displaceably or pivotally arranged **in that** the holding element is displaceable or pivotable on the longitudinal edge (21) of the first half-shell (13).

4. The device according to one of the preceding claims 2 or 3, **characterized in that** the holding element is connected with the first half-shell (13) via a second joint (47), which is formed, for example, as a hinge, in particular as a film hinge.

5. The device according to one of the preceding claims 2 to 4, **characterized in that** a holding element support (63), preferably with an engagement groove (56), is provided on the longitudinal edge (23) of the second half-shell (15).

6. The device according to one of the preceding claims 2 to 5, **characterized in that** upon closing of the two half-shells (13, 15), the holding element slides over the longitudinal edge (23) of the second half-shell (15) out of the closing chamber (46).

7. The device according to one of the preceding claims 2 to 6, **characterized in that** in the closed position of the half-shells (13, 15), the holding element is clamped between the edges of the half-shells.

8. The device according to one of the preceding claims 2 to 7, **characterized in that** in the closed position, the holding element is located substantially outside the enclosed chamber (39), and preferably between the wings (25, 27).

9. The device according to one of the preceding claims 2 to 8, **characterized in that** a plurality of holding elements, in particular two arms, are present, wherein each holding element supports at least one stopper (59), in particular a first stopper for the front haptic of the lens and a second stopper for the rear haptic of the lens (33).

10. The device according to one of the preceding claims 1 to 9, **characterized in that** each half-shell (13, 15) is equipped with a support (35, 37), formed, for instance, as slide rails.

11. The device according to one of the preceding claims 1 to 10, **characterized in that** wings (25, 27) are arranged on both half-shells (13, 15), in particular on the longitudinal edge (21, 23) of the half shells (13, 15).

12. The device according to the preceding claim 11, **characterized in that** a closure (73), in particular a snap closure, is formed on the wings (25, 27).

13. The device according to one of the preceding claims 1 to 12, **characterized in that** a plug device (73) is formed on one of the half-shells (13, 15) for inserting into a receiving opening of an injector housing (1).

14. The device according to one of the preceding claims 1 to 13, **characterized in that** this is formed as a cartridge for insertion into an injector, in particular an injector housing (1).

15. An injector with an injector housing (1) and a plunger (9), with a device, according to anyone of claims 1 to 13, which is formed as a cartridge.

## Revendications

1. Dispositif pour le logement d'une lentille intraoculaire (33) avec une première demie coque (13, 15) et une seconde demie coque (13, 15) qui sont reliées de manière articulée l'une à l'autre par une première articulation (29) et qui peuvent être déplacées l'une par rapport à l'autre d'une position ouverte à une position fermée, cependant que
- les demies coques (13, 15) forment, dans la position ouverte, un compartiment ouvert (46) qui sert à insérer une lentille et
- les demies coques (13, 15) forment, dans la position fermée, un compartiment fermé (39) qui définit un passage d'éjection et qui sert à éjecter la lentille,
**caractérisé en ce**
**qu'**au moins un arrêt (59) est placé déplaçable ou pivotant sur la première des deux demies coques (13), arrêt qui délimite, dans la position ouverte, le compartiment ouvert (46) dans le sens de l'éjection et qui est positionné, dans la position fermée, sensiblement à l'extérieur du compartiment fermé (39) à côté du passage d'éjection.

2. Dispositif selon la revendication 1, **caractérisé en ce que** l'arrêt est configuré sur un élément de retenue, par exemple un élément de couvercle (45) ou un bras, qui surmonte ou enjambe, dans la position ouverte, le compartiment ouvert (46) et qui est positionné, dans la position fermée, sensiblement à l'extérieur du compartiment fermé (39).

3. Dispositif selon la revendication 2, **caractérisé en ce que** l'arrêt est placé déplaçable ou pivotant **en ce que** l'élément de retenue est placé déplaçable ou pivotant sur le bord longitudinal (21) de la première demie coque (13).

4. Dispositif selon l'une des revendications précédentes 2 ou 3, **caractérisé en ce que** l'élément de retenue est relié à la première demie coque (13) par une seconde articulation (47) qui est, par exemple, configurée comme une charnière, en particulier comme une charnière élastique.

5. Dispositif selon l'une des revendications précédentes 2 à 4, **caractérisé en ce qu'**un support d'élément de retenue (63), de préférence avec une rainure d'encliquetage (56), est configuré sur le bord longitudinal (23) de la seconde demie coque (15).

6. Dispositif selon l'une des revendications précédentes 2 à 5, **caractérisé en ce que**, lors de la fermeture des deux demies coques (13, 15), l'élément de retenue glisse hors du compartiment qui se ferme (46) par dessus le bord longitudinal (23) de la seconde demie coque (15).

7. Dispositif selon l'une des revendications précédentes 2 à 6, **caractérisé en ce qu'**en position fermée des demies coques (13, 15) l'élément de retenue est coïncé entre les bords des demies coques.

8. Dispositif selon l'une des revendications précédentes 2 à 7, **caractérisé en ce qu'**en position fermée l'élément de retenue se situe sensiblement à l'extérieur du compartiment fermé (39) et de préférence entre des ailettes (25, 27).

9. Dispositif selon l'une des revendications précédentes 2 à 8, **caractérisé en ce qu'**il existe plusieurs éléments de retenue, en particulier deux bras, chaque élément de retenue portant au moins un arrêt (59), en particulier un premier arrêt pour la partie haptique antérieure de la lentille et un second arrêt pour la partie haptique postérieure de la lentille (33).

10. Dispositif selon l'une des revendications précédentes 2 à 9, **caractérisé en ce que** chaque demie coque (13, 15) est équipée d'un support (35, 37), par exemple configuré comme des glissières.

11. Dispositif selon l'une des revendications précédentes 2 à 10, **caractérisé en ce que** des ailettes (25, 27) sont placées sur les deux demies coques (13, 15), en particulier sur le bord longitudinal (21, 23) des demies coques (13, 15).

12. Dispositif selon la revendication précédente 11, **caractérisée en ce qu'**une fermeture (73), en particulier une fermeture à cliquet, est configurée sur les ailettes (25, 27).

13. Dispositif selon l'une des revendications précédentes 1 à 12, **caractérisé en ce qu'**un dispositif d'enfichage (73) est configuré sur l'une des demies coques (13, 15) pour enficher dans une ouverture de logement d'un boîtier d'injecteur (1).

14. Dispositif selon l'une des revendications précédentes 1 à 13, **caractérisé en ce que** celui-ci est configuré comme une cartouche à mettre en place dans un injecteur, en particulier dans un boîtier d'injecteur (1).

15. injecteur avec un boîtier d'injecteur (1) et un piston déplaçable en longueur dans le boîtier d'injecteur avec un dispositif configuré comme une cartouche selon l'une des revendications 1 à 13.
